# EUROPEAN PATENT APPLICATION

(11) **EP 3 385 699 A1**
(43) Date of publication of application: **10.10.2018**
(21) Application number: 17382192.7
(22) Date of filing: 07.04.2017
(51) Int. Cl.: G01N 15/14, G01N 15/10, B01L 3/00, C12N 5/09, G01N 21/53, G01N 21/64, G01N 21/65, G01N 33/574

(54) **OPTOFLUIDIC DEVICE AND METHOD FOR DETECTING CIRCULATING TUMOUR CELLS**

(71) Applicant: Universitat Rovira i Virgili, 43003 Tarragona (ES); Fundació Privada Institució Catalana de Recerca I Estudis Avançats, 08010 Barcelona (ES); Fundacio Privada Centre Tecnologic de la Quimica de Catalunya, 43007 Tarragona (ES); Fundacion de Investigacion HM Hospitales, 28015 Madrid (ES); Medcom Tech, S.A., 28042 Madrid (ES); MEDCOM ADVANCE, S.A., 08029 Barcelona (ES)
(72) Inventor: ÁLVAREZ-PUEBLA, Ramón A., E-43007 Tarragona (ES); PEDROL, Eric, E-43007 Tarragona (ES); MASSONS, Jaume, E-43007 Tarragona (ES); DÍAZ, Francisco, E-43007 Tarragona (ES); GARCÍA ALGAR, Manuel, E-43760 El Morell-Tarragona (ES); NAZARENUS, Moritz Julian, E-43007 Tarragona (ES); GARCÍA-RICO FERNÁNDEZ, Eduardo, E-28015 Madrid (ES)
(74) Representative: ABG Patentes, S.L.

(57) **Abstract**

The present invention relates to a microfluidic device for the detection of circulating tumour cells (CTCs) in a fluid sample. The invention also relates to an *in vitro* method for the detection and/or quantification of circulating tumour cells (CTCs) in a fluid sample. Further, the invention relates to *in vitro* methods for (a) diagnosing a tumour and/or metastasis in a subject, and (b) determining the prognosis of a subject suffering from a tumour and/or metastasis. Finally, the invention refers to a kit comprising (a) a microfluidic device according to the first aspect of the invention, and (b) labelled probes targeting a first CTCs surface marker and, optionally, at least a second CTCs surface marker.

## Description

### TECHNICAL FIELD OF THE INVENTION

The present invention relates to the field of cancer. In particular, the invention relates to the field of devices and methods for the detection of circulating tumour cells.

### BACKGROUND OF THE INVENTION

Prognosis and assessment of breast cancer treatment is established at the time of diagnosis by pathologic examination, immunohistochemistry and molecular biology after core needle biopsy or surgical specimen extraction. However, in metastatic cancer (stage IV), the knowledge of the current health state of the patient at different times of the disease evolution is crucial for treatment efficacy. Unfortunately, repetition of the biopsy is very invasive and not always feasible. Thus, to overcome this situation, several methods for the detection of circulating tumour cells (CTCs) in blood (i.e. liquid biopsy) have been proposed in recent years showing diagnostic and prognostic value. However, CTCs are found in extremely low concentrations in blood samples: a normal concentration in a human cancer patient is approximately 1-100 CTCs per mL of blood. Currently, most of these liquid biopsy methods are based on the use of the epithelial cell adhesion molecule (EpCAM) antibodies to capture and enrich the CTC fraction with the subsequent characterisation with immunostaining or retro-transcription polymerase chain reaction (CellSearch® CTC test, www.cellsearchctc.com). Other methods employ the intrinsic differences in shape, size and/or density of the tumour cells as compared with the normal circulating cells to collect and later recount CTCs by using filters, activated surfaces or particles. Many of these cell enrichment methods are performed in microfluidic devices (K.-A. Hyun, et al., Microfluidic flow fractionation device for label-free isolation of circulating tumor cells (CTCs) from breast cancer patients. Biosensor Bioelectron 40, 206-212, 2013; Peng Li et al., Probing circulating tumor cells in microfluidics, Lab Chip. 2013 February 21; 13(4): 602-609).

Regarding the direct identification of CTCs in a sample without cell enrichment, several alternatives mainly based in flow-cytometry (H. Ulrich and A. Tárnok, Flow cytometry detection of circulating tumor cells: Achievements and limitations as prognostic parameters. Cytometry A 85, 201-202, 2014) have been proposed based on fluorescence (M. Watanabe, et al., Multicolor detection of rare tumor cells in blood using a novel flow cytometry-based system, Cytometry A 85, 206-213, 2014) or ultrasounds (E. M. Strohm and M. C. Kolios, Classification of blood cells and tumor cells using label-free ultrasound and photoacoustics, Cytometry A 87, 741-749, 2015). These later approaches, however, require of expensive instrumental techniques which hinder the generalised application in the daily clinical practice at the oncology point of care.

Accordingly, there is a need in the art for the development of alternative devices and methods for the prognosis and assessment of cancer patients by directly detecting CTCs in blood that solves the problems associated with current techniques.

### SUMMARY OF THE INVENTION

Thus, in a first aspect, the invention relates to a microfluidic device for the detection of circulating tumour cells (CTCs) in a fluid sample comprising:
(a) a cell focusing element selected from a hydrodynamic focusing element and an inertial focusing element, wherein
   the hydrodynamic focusing element comprises at least three inlets, a first central inlet for the entrance of a labelled fluid sample, and a second and third inlets for the entrance of a focusing fluid for flow focusing of the labelled fluid sample in a first plane, wherein the second and third inlets are situated at opposite sides of the first central inlet, and optionally, a fourth and fifth inlets for the entrance of a focusing fluid for flow focusing of the labelled fluid sample in a second plane perpendicular to the first plane, wherein the fourth and fifth inlets are situated at opposite sides of the first central inlet, all the inlets converging into a single microfluidic channel; and wherein
   the inertial focusing element comprises a functional microchannel selected from the group consisting of a straight channel, a straight channel with pillar arrays, a straight channel with expansion-contraction arrays, a spiral channel, a serpentine channel, and combinations thereof;
(b) a single microfluidic channel comprising at least two interrogation regions, wherein each interrogation region comprises at least one waveguide for labelled fluid sample excitation, and at least one waveguide for signal detection, wherein the at least one waveguide for signal detection is not placed at a 180 degree angle with respect to the at least one waveguide for sample excitation, wherein the microfluidic channel optionally comprises lenses and/or mirrors for ensuring an adequate path for radiation along the interrogation region; and
(c) at least one outlet for the exit of the labelled sample and focusing fluids.

In a second aspect, the invention relates to an *in vitro* method for the detection of circulating tumour cells (CTCs) in a fluid sample, wherein the method comprises the steps of:
(a) incubating the fluid sample with a labelling solution wherein the labelling solution comprises a first labelled probe targeting a first CTCs surface marker and at least a second labelled probe targeting a second CTCs surface marker;
(b) removing the excess of any labelled probe not bound to the CTCs, thereby obtaining a labelled fluid sample; and
(c) detecting in a microfluidic device according to the first aspect of the invention a first signal corresponding to the first labelled probe and at least a second signal corresponding to the at least second labelled probe, in the labelled fluid sample;
wherein the detection of the first signal and the second signal is indicative of the presence of CTCs in the fluid sample; wherein the first and second probes are labelled with a different label; wherein the first and second labels are independently selected from the group consisting of a fluorescent label, an elastic radiation label, and a Raman label; and wherein the first and second probes are independently selected from the group consisting of an antibody, an antibody derivative, a lectin and an aptamer.

In a third aspect, the invention also relates to an *in vitro* method for the detection of circulating tumour cells (CTCs) in a fluid sample, wherein the method comprises the steps of:
(a) incubating the fluid sample with a labelling solution wherein the labelling solution comprises a first labelled probe targeting a first CTCs surface marker and at least a second labelled probe targeting a second CTCs surface marker, wherein the first and second probes are fluorescently labelled; or alternatively incubating the fluid sample with a labelling solution wherein the labelling solution comprises a first labelled probe targeting a first CTCs surface marker, wherein the first probe is fluorescently labelled;
(b) removing the excess of any labelled probe not bound to the CTCs, thereby obtaining a labelled fluid sample; and
(c) detecting in a microfluidic device according to the first aspect of the invention a first signal corresponding to the first labelled probe and at least a second signal selected from the group consisting of the at least second labelled probe, autofluorescence, and combinations thereof, in the labelled fluid sample;
wherein the detection of the first signal and the second signal is indicative of the presence of CTCs in the fluid sample; wherein the first and second probes are labelled with a different label; wherein the first and second labels are independently selected from the group consisting of a fluorescent label, an elastic radiation label, and a Raman label; and wherein the first and second probes are independently selected from the group consisting of an antibody, an antibody derivative, a lectin and an aptamer.

In a fourth aspect, the invention further relates to an *in vitro* method for diagnosing a tumour and/or metastasis in a subject, wherein the method comprises the step of detecting the presence of CTCs in a fluid sample from said subject by a method according to the second or third aspects of the invention; wherein the detection of the presence of CTCs in the fluid sample is indicative that the subject suffers from a tumour and/or metastasis.

In a fifth aspect, the invention relates to an *in vitro* method for quantifying circulating tumour cells (CTCs) in a fluid sample, wherein the method comprises the steps of:
(a) detecting the presence of CTCs in a fluid sample from said subject by a method according to the second or third aspects of the invention; and
(b) quantifying the number of CTCs present in the fluid sample from said subject.

In a sixth aspect, the invention relates to an *in vitro* method for determining the prognosis of a subject suffering from a tumour and/or metastasis, wherein the method comprises the steps of:
(a) detecting the presence of CTCs in a fluid sample from said subject by a method according to the second or third aspects of the invention;
(b) quantifying the number of CTCs present in the fluid sample from said subject; and
(c) comparing the number of CTCs present in the fluid sample from said subject with the number of CTCs present in a reference sample;
wherein an increase in the number of CTCs in the fluid sample with respect to the number of CTCs in the reference sample is indicative that the subject has a poor prognosis, or wherein a decrease in the number of CTCs in the fluid sample with respect to the number of CTCs in the reference sample is indicative that the subject has a good prognosis.

In a seventh aspect, the invention relates to a kit comprising:
(a) a microfluidic device according to the first aspect of the invention, and
(b) a first labelled probe targeting a first CTCs surface marker and, optionally, at least a second labelled probe targeting a second CTCs surface marker
wherein the first and second probes are labelled with a different label and wherein the first and second labels are independently selected from the group consisting of a fluorescent label, an elastic radiation label, and a Raman label; and wherein the first and second probes are independently selected from the group consisting of an antibody, an antibody derivative, a lectin and an aptamer.

### DESCRIPTION OF THE FIGURES

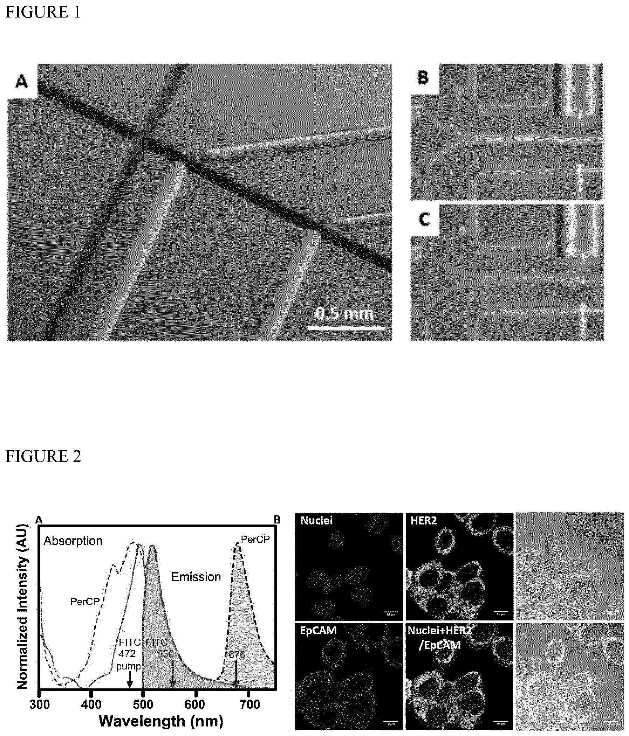

**FIGURE 1****. Optofluidic chip. (A)** SEM image of the low-cost micro-optofluidic chip which comprises a 3D- flow focusing channel for the single cell alignment and several integrated optical fibres for excitation (473 nm) and collection. **(B)** and **(C)** The use of a pressure controller allows for an accurate positioning of the 3D focused sample relative to the pumping light beam; a blue light path was visible only when the sample focused flow intersected the pumping beam -probably caused by scattered light from the sample saline solution-. As soon as the height of the focused sample was increased or lowered for a given distance the light path disappeared and no signal was detected in the detectors.
**FIGURE 2****. Absorption and emission properties of the dyes used to label the antibodies and their performance on the cell membrane. (A)** Absorption and emission profiles of anti-her2 FITC conjugated and anti-EpCAM PerCP/Cy5.5 conjugated antibodies. **(B)** Laser scanning confocal microscopy images of AU-565 cells stained with anti-Her2 FITC conjugated and anti-EpCAM PerCP/Cy5.5 conjugated antibodies. Nuclei were stained with DAPI. Scale bar 10 mm

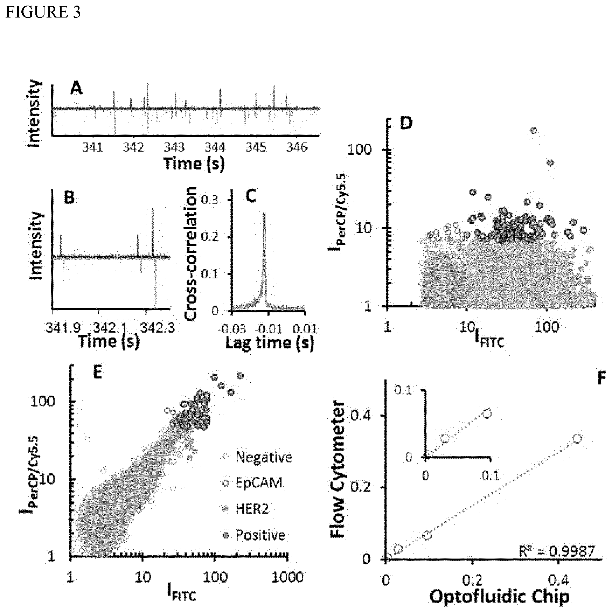

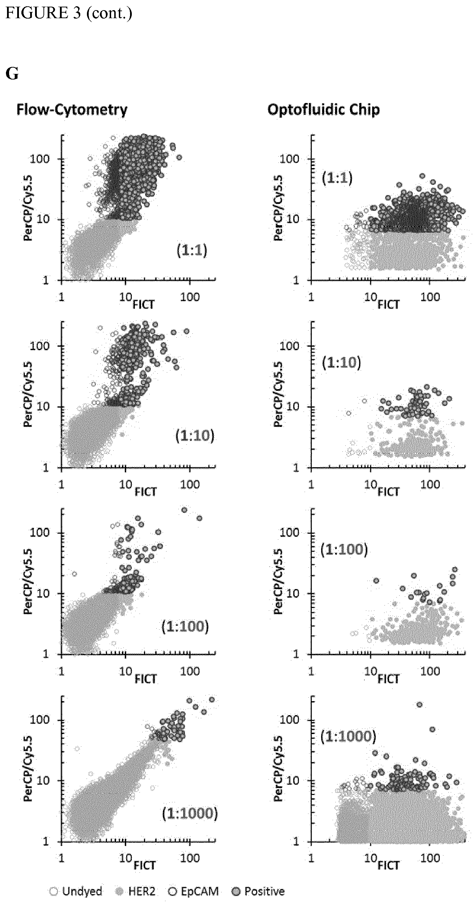

**FIGURE 3****. Signal recording in the optofluidic chip.** (**A**) A short record of the fluorescence signals obtained for the 1:10 sample. **(B)** Cross-correlogram of the green and red fluorescence signals. **(C)** Delay (Δt=-0.0119 s) between the read and the green signals. Results obtained with the **(D)** optofluidic chip and **(E)** flow cytometer for a sample containing 1 AU-565 (HER2 positive/EpCAM positive) per 1000 RAMOS cells (HER2 negative/EpCAM negative). **(F)** Comparison between the optofluidic chip and the flow cytometry cell ratios of 1:1, 1:10, 1:100 and 1:1000 Au-565:RAMOS. (G) Results obtained with the flow cytometer and the optofluidic chip for cell ratios of 1:1, 1:10, 1:100 and 1:1000 Au-565:RAMOS.

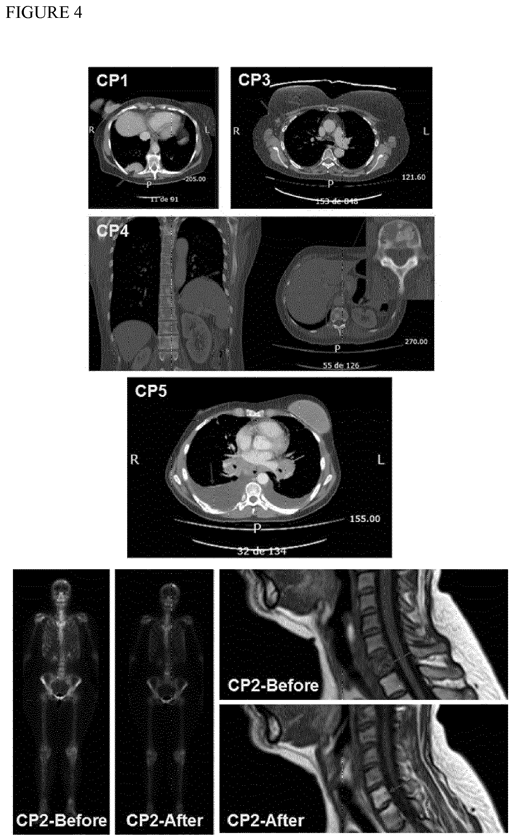

**FIGURE 4****. Computerized tomography of the cancer patients at the time of blood extraction.** CP1, lung metastasis. CP3, recently diagnosed and untreated yet metastatic adenopathy. CP4, vertebral osteoblastic metastasis showing the current bone progression of the disease. CP5, malignant pleural effusion and mediastinal mass. At the time of the study, she had already been given four cycles of specific treatment. CP2, bone scintigraphy and MRI before (showing a lytic metastasis in C6) and at the time of blood extraction (complete remission).

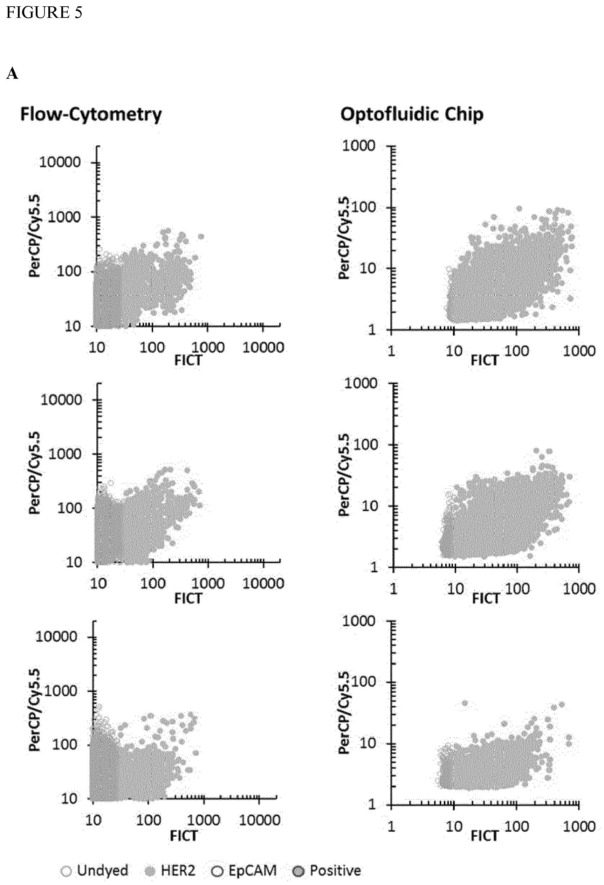

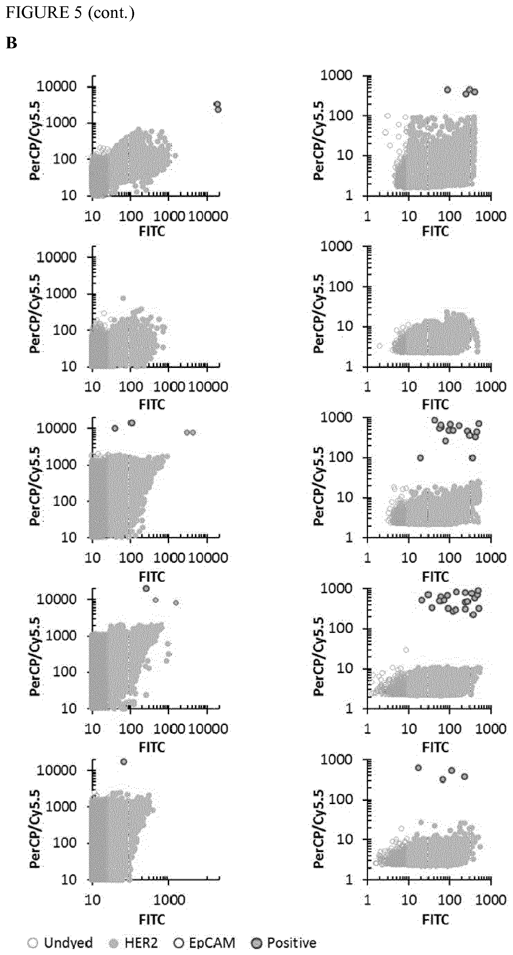

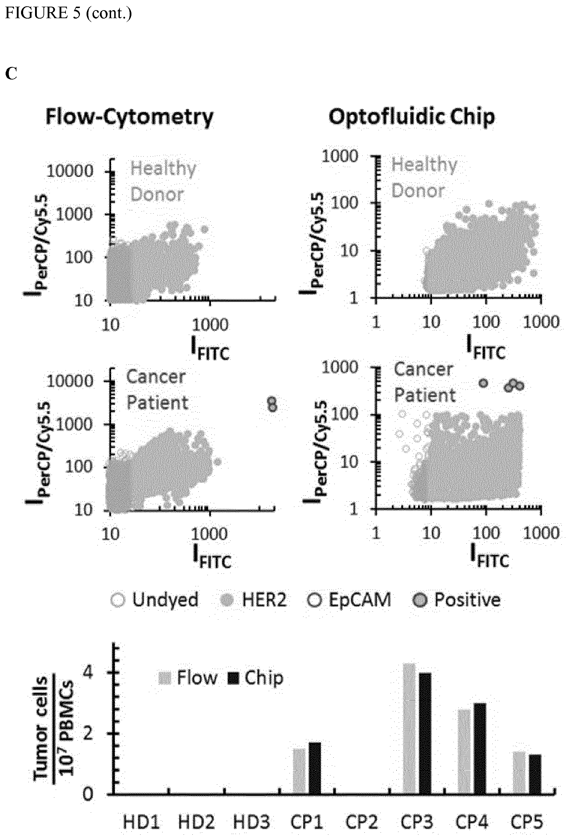

**FIGURE 5****. Flow-cytometry Vs. optofludics.** (**A**) Results obtained with the flow cytometer and the optofluidic chip for the blood samples from healthy donors. (B) Results obtained with the flow cytometer and the optofluidic chip for the blood samples of cancer patients. (C) Comparison between the results obtained with the flow cytometer and those with the optofluidic device for healthy donors and cancer patients.

### DETAILED DESCRIPTION OF THE INVENTION

The inventors have designed a microfluidic device for the fluorescence quantification of circulating tumour cells (CTCs) in real samples wherein the device comprises a cell focusing element and wherein all optics are integrated into the chip. To test its performance in the detection of CTCs, two cell membrane targets, the epithelial cell adhesion molecule, EpCAM, and the receptor tyrosine-protein kinase, HER2, are selected to recognise circulating tumour cells of HER2 positive breast cancer. This particular malignancy was chosen because it is an aggressive tumour, linked with up to the 30% of the total breast cancers. The efficiency of the optofluidic platform was demonstrated on cell lines and in a variety of healthy donors and metastatic-breast cancer patients. The inventors compare their results with those of state of the art diagnostic tools and flow-cytometry.

### A microfluidic device for the detection of circulating tumour cells (CTCs)

In a first aspect, the invention relates to a microfluidic device for the detection of circulating tumour cells (CTCs) in a fluid sample comprising:
(a) a cell focusing element selected from a hydrodynamic focusing element and an inertial focusing element, wherein
   the hydrodynamic focusing element comprises at least three inlets, a first central inlet for the entrance of a labelled fluid sample, and a second and third inlets for the entrance of a focusing fluid for flow focusing of the labelled fluid sample in a first plane, wherein the second and third inlets are situated at opposite sides of the first central inlet, and optionally, a fourth and fifth inlets for the entrance of a focusing fluid for flow focusing of the labelled fluid sample in a second plane perpendicular to the first plane, wherein the fourth and fifth inlets are situated at opposite sides of the first central inlet, all the inlets converging into a single microfluidic channel; and wherein
   the inertial focusing element comprises a functional microchannel selected from the group consisting of a straight channel, a straight channel with pillar arrays, a straight channel with expansion-contraction arrays, a spiral channel, a serpentine channel, and combinations thereof;
(b) a single microfluidic channel comprising at least two interrogation regions, wherein each interrogation region comprises at least one waveguide for labelled fluid sample excitation, and at least one waveguide for signal detection, wherein the at least one waveguide for signal detection is not placed at a 180 degree angle with respect to the at least one waveguide for sample excitation, wherein the microfluidic channel optionally comprises lenses and/or mirrors for ensuring an adequate path for radiation along the interrogation region; and
(c) at least one outlet for the exit of the labelled sample and focusing fluids.

The expression "microfluidic device", as used herein, refers to a system in which low volumes of fluids are processed to achieve multiplexing, automation and high-throughput screening. In a preferred embodiment, the microfluidic device is fabricated with a polymeric material. The term "polymeric material", as used herein, refers to any organic compound, natural or synthetic, with high molecular weight made of repetitive structural units, preferably to elastomer and thermoplastic polymers. Exemplary polymeric materials that can be used for the present invention are, without limitation, polydimethylsiloxane (PDMS), poly(methylmethacrylate) (PMMA), polycarbonate (PC), poly(ethylene terephthalate) glycol (PETG), polyethylene (branched) (LDPE), polystryrene (PS), polypropylene (PP), polyetheretherketone (PEEK), polyethylene terephthalate (PET), polyethylene (PE), polyvinylidene chloride (PVDC), polyvinyl chloride (PVC), polysulfone (PSU), cyclic olefin polymer (COP) and cyclic olefin copolymer (COC). In a preferred embodiment the polymeric material is polydimethylsiloxane (PDMS), preferably PDMS sealed with glass. In a more preferred embodiment, the polymeric material is PDMS sealed with PDMS.

The term "circulating tumour cell" or "CTC", as used herein, refers to a cell that has shed into the vasculature from a primary tumour and circulates in the bloodstream. The term CTC, in the context of the present invention, also includes tumour cells in the peritoneal and lymphatic system. CTCs can be identified by methods well known by the person skilled in the art, for example by using specific antibodies able to recognise specific tumour markers such as EpCAM, HER2, CK19 or PSA. The term "tumour cell" or "TC", as used herein, refers to a cell that can grow and divide at an unregulated, quick pace. TC can be identified by methods well known by the person skilled in the art, for example by using specific antibodies able to recognise specific tumour markers such as EpCAM, HER2, CK19 or PSA.

The expression "fluid sample", as used herein, refers to any kind of liquid sample that potentially contains TCs or CTCs. In a preferred embodiment, the fluid sample is a biofluid. The term "biofluid" in the context of the present invention refers to any biological secretion or fluid, whether physiological or pathological, which is produced in the body of a subject. Such biofluids include, without limitation, blood, bronchoalveolar washing fluid, urine, nasal secretion, ear secretion, urethral secretion, cerebrospinal fluid, pleural fluid, synovial fluid, peritoneal fluid, ascites fluid, pericardial liquid, amniotic fluid, gastric juice, lymphatic fluid, interstitial fluid, saliva, sputum, liquid deposition, tears, mucus, sweat, milk, semen, vaginal secretions, fluid coming from ulcer, blisters, abscesses and other surface eruptions. Said samples can be obtained by conventional methods, using processes known in the state of art by the person skilled in the art, such as blood extraction, instillation and aspiration of liquid during bronchofibroscopy, cisternal, ventricular or lumbar puncture, pleural puncture or thoracocentesis, joint or synovial percutaneous puncture, abdominal puncture, amniocentesis, expectoration, peritoneal percutaneous puncture, pericardial percutaneous puncture, etc., or by simple harvesting. In a preferred embodiment the biofluid is blood, particularly whole blood. The blood sample is typically extracted by means of puncturing an artery or vein, normally a vein from the inner part of the elbow or from the back of the hand, the blood sample being collected in an air-tight vial or syringe. A capillary puncture normally on the heel or on the distal phalanxes of fingers can be performed for analysis by means of a micromethod. Typically when the liquid sample is blood, the first method of the invention detects CTC and when the liquid sample is other biofluid different from blood, the method detects TC.

The micro fluidic device of the invention comprises a cell focusing element. By focusing the cell-carrying fluid in a small volume, the possibility of multiple detection of a single cell in the measurement area is minimised and/or prevented. Focusing ensures that the cell-carrying fluid volume does not contain more than one cell at a time when it crosses the interrogation region. Cell focusing can be achieved by hydrodynamic focusing and/or inertial focusing.

The term "hydrodynamic focusing", as used herein, refers to an effect achieved with multiple flows with substantially different flow rates come into contact, resulting in the streamlining of a central core stream, which is pinched between lateral sheath streams. Precise control of flow rates by multiple pumps and/or pressure sources allow positioning the central core stream in a desired flow direction. In a preferred embodiment, the flow rate of the solution for cell focusing is the same or higher than the flow rate of the cell solution. Preferably the flow rate of the focusing fluid is between 0.005 and 50 mL/h and the flow rate of the cell solution is between 0.005 and 50 ml/h. In another embodiment, the focusing fluid is selected from the group consisting of a cell culture medium, a buffer solution and water. In a more preferred embodiment the solution is a buffer solution, preferably selected from the group consisting of phosphate-buffered saline (PBS), trizma buffer, 4-(2-hydroxyethyl)-1-piperazineethanesulfonic acid (HEPES), 2-(N-morpholino) ethanesulfonic acid (MES), Tris buffer, 4-(N-morpholino) butanesulfonic acid (MOBS), sodium acetate, citric acid, and any combination thereof. In another preferred embodiment, the solution for cell focusing is a cell culture medium, preferably selected from the group consisting of DMEM, EMEM, IMDM, RPMI-1640, GMEM, BME, MEM and Medium 199. In a preferred embodiment, the simultaneous control of sheath fluid flow from two independent lateral channels is used to horizontally constrict the focused central core fluid in order to position it (up / down) within the fluidic channel. In another preferred embodiment, the simultaneous control of sheath fluid flow from four independent lateral channels is used to vertically and horizontally constrict the focused central core fluid in order to position it (up / down + left / right) within the fluidic channel. Strictly speaking, the cells themselves are not being focused. Instead, the fluid containing these cells is spatially restricted to a section of finite size taking advantage of the fluid flow properties at these small scales, wherein the low Reynolds number will keep the fluid in a laminar state (Re<<1). The relative position of the cells in the microfluidic channel before it is laterally and vertically compressed will be maintained once the fluid section decreases to a given size, whereby the cells occupy positions which are generally not contained in a single trajectory but are distributed randomly in a cross section, albeit a much smaller cross section than before compressing the flow that carries the cells.

In the hydrodynamic focusing element of the invention the first, second and third inlets are situated at the same plane (first plane). Additional inlets can be situated at the first plane. In a preferred embodiment, the hydrodynamic focusing element also contains a fourth and fifth inlets both situated at the same plane (second plane) but this second plane being perpendicular to the first plane.

The term "labelled fluid sample", as used herein, refers to a fluid sample which has been incubated with a "labelling solution", as defined in the context of the second aspect of the invention.

The term "inertial focusing", as used herein, refers to an effect achieved with fluid flows working in the intermediate Reynolds number range (∼1< Re< ∼100). In this intermediate range, inertial and fluid viscosity effects are not negligible and bring about several intriguing effects that form the basis of inertial microfluidics including (i) inertial migration and (ii) secondary flow in curved channels (Zhang et al., Fundamentals and applications of inertial microfluidics: a review, Lab Chip, 2016, 16, 10). These effects can be harnessed depending on the particular design of the geometry of the microchannels and can be used for cell sample processing (i.e. to position the cell fluid sample in a desired flow direction). In a particular embodiment, the inertial focusing element of the invention comprises a microchannel selected from the group consisting of a straight channel, a straight channel with pillar arrays, a straight channel with expansion-contraction arrays, a spiral channel, a serpentine channel, and combinations thereof (Jun Zhang et al., Fundamentals and applications of inertial microfluidics: a review, Lab Chip, 2016, 16, 10). In the case of inertial focusing, a single flow may be sufficient to achieve the desired focusing effect. Thus, no additional channels are needed to compress the fluid. Above a given flow rate, the inertial effects of a cell-carrying fluid contained within a microfluidic channel begin to be relevant to the extent that the forces involved can displace the cells within the channel to an equilibrium trajectory as they move through the microchannel. Unlike conventional hydrodynamic focusing, these trajectories are fixed (at least in one dimension of the channel) to a constant position. This effect allows positioning the cells in an optimal direction for improved signal acquisition as the cells cross the interrogation zones. As an additional advantage, the use of a single input instead of using five inputs means that the sample is not diluted with focusing sheath fluid, which may allow the serial connection of microfluidic chips to analyse the same sample.

The term "interrogation region", as used herein, refers to a portion of the microchannel which comprises at least one waveguide for labelled fluid sample excitation, and at least one waveguide for signal detection. The at least one waveguide for labelled fluid sample excitation allows the interrogation region to be illuminated with an electromagnetic radiation of a suitable wavelength, such as visible light, elastic or inelastic (Raman) radiation. The cells of the labelled fluid sample pass the interrogation region such that positive CTCs are identified by the simultaneous detection of specific CTC markers. The detection of specific CTC markers is achieved by means of suitable labelled probes as defined in the context of the second aspect of the invention. The single microfluidic channel can contain at least two, at least three, at least four, at least five, at least six, at least seven, at least eight, at least nine, at least ten or more interrogation regions.

The at least two interrogation regions of the device according to the first aspect of the invention allow for signal correlation analysis; wherein the detection of a single event (registration of a peak of intensity) in only one interrogation region is not sufficient to be accounted as a cell detection event. A cell detection event is only assigned when one given cell is detected in two independent interrogation regions so that the involved signals are correctly correlated by a pairing algorithm, as described in the accompanying examples. This methodology greatly increases the confidence as it discards possible false positive events in measurements were the detection of very few positive events is to be expected. By introducing this self-imposed pairing requirement between signals, some events may be rejected, but the detection threshold is lower than that corresponding to a single interrogation region. The pairing algorithm is also capable of rejecting positive events wherein the detected intensity-versus-time peaks are too wide, and which can be generally related to multiple cell detection. This is because the size of the excitation spot is of the order of one single cell (the smaller the excitation spot, the bigger the relative change in width of the detected peak when an aggregate of cells passes by the interrogation region). In the context of the present invention, the term "excitation spot" correspond to the exact volume of the interrogation region which is illuminated with an electromagnetic radiation of a suitable wavelength.

The term "waveguide", as used herein, refers to a structure that directs electromagnetic waves. Some of the most common types of waveguides include rectangular waveguides and optical fibres, flat waveguides and photonic crystals. Waveguides can be incorporated into silica, PDMS or liquid core. Waveguides can be constructed to carry waves over a wide portion of the electromagnetic spectrum. They are especially useful in the optical spectrum. The optical spectrum, also known as the visible spectrum, corresponds to the portion of the electromagnetic spectrum that comprises wavelengths from about 390 to 700 nm. Electromagnetic radiation in this range of wavelengths is called visible light or simply light.

In an embodiment, the at least one waveguide for labelled fluid sample excitation, and at least one waveguide for signal detection within one interrogation region are of the same type. In an embodiment, the at least one waveguide for labelled fluid sample excitation, and at least one waveguide for signal detection within one interrogation region are of a different type. In an embodiment, the excitation and detection waveguides in the at least two interrogation regions are of the same type. In an embodiment, the excitation and detection waveguides in the at least two interrogation regions are of a different type.

In an embodiment, the at least one waveguide for signal detection is placed at a 175 degree, at a 170 degree, at a 165 degree, at a 160 degree, at a 155 degree, at a 150 degree, at a 145 degree, at a 140 degree at a 135 degree, at a 130 degree, at a 125 degree, at a 120 degree, at a 115 degree, at a 110 degree, at a 105 degree, at a 100 degree, at a 95 degree, at a 90 degree, at a 85 degree, at a 70 degree, at a 65 degree, at a 60 degree, at a 55 degree, at a 50 degree, at a 45 degree, at a 40 degree, at a 35 degree, at a 30 degree, at a 25 degree, at a 20 degree, at a 15 degree, at a 10 degree, or at a 5 degree angle with respect to the at least one waveguide for sample excitation.

In a preferred embodiment, the at least one waveguide for signal detection is placed at a 45 or at a 135 degree angle with respect to the at least one waveguide for sample excitation.

In a preferred embodiment, the at least one waveguide for labelled fluid sample excitation and the at least one waveguide for signal detection are optical fibres. The term "optical fibre", as used herein, refers to flexible transparent fibre of extremely pure glass or plastic, generally between 10 and 200 microns in diameter, through which light can be transmitted by successive internal reflections.

In a preferred embodiment, the microfluidic channel in the device of the invention optionally comprises lenses and/or mirrors for ensuring an adequate light path along the interrogation region. Lenses and/or mirrors which would be suitable for use in the device of the invention are known in the art (Ibarlucea et al., Disposable multiple internal reflection systems for photonic cell screening, 14th International Conference on Miniaturized Systems for Chemistry and Life Sciences 3 - 7 October 2010, Groningen, The Netherlands).

In a preferred embodiment of the device of the invention, the cell focusing element is a hydrodynamic focusing element and the waveguides are optical fibres.

### An in vitro method for the detection of circulating tumour cells (CTCs)

In a second aspect, the invention relates to an *in vitro* method for the detection of circulating tumour cells (CTCs) in a fluid sample, wherein the method comprises the steps of:
(a) incubating the fluid sample with a labelling solution wherein the labelling solution comprises a first labelled probe targeting a first CTCs surface marker and at least a second labelled probe targeting a second CTCs surface marker;
(b) removing the excess of any labelled probe not bound to the CTCs, thereby obtaining a labelled fluid sample; and
(c) detecting in a microfluidic device according to the first aspect of the invention a first signal corresponding to the first labelled probe and at least a second signal corresponding to the at least second labelled probe, in the labelled fluid sample;
wherein the detection of the first signal and the second signal is indicative of the presence of CTCs in the fluid sample; wherein the first and second probes are labelled with a different label; wherein the first and second labels are independently selected from the group consisting of a fluorescent label, an elastic radiation label, and a Raman label; and wherein the first and second probes are independently selected from the group consisting of an antibody, an antibody derivative, a lectin and an aptamer.

The term "circulating tumour cell" or "CTC" and "fluid sample" have been defined in the context of the first aspect of the invention.

The method according to the second aspect of the invention does not involve a prior step of enrichment of the CTC fraction in the blood sample (i.e. the CTCs are directly detected in the blood sample).

Step (a) of the first method of the invention comprises incubating the fluid sample with a labelling solution wherein the labelling solution comprises a first labelled probe targeting a first CTCs surface marker and at least a second labelled probe targeting a second CTCs surface marker.

The expression "labelling solution", as used herein, refers to a solution comprising at least two labelled probes. The expression "labelled probe" refers to a probe that targets a CTCs surface marker that is labelled. In a preferred embodiment, the labelling solution comprises at least two labelled probes, at least three labelled probes, at least four labelled probes, at least five labelled probes, at least six labelled probes, at least seven labelled probes, at least eight labelled probes, at least nine labelled probes, at least ten labelled probes or more targeting CTCs surface markers, preferably targeting different CTCs surface markers.

The probes capable of targeting a CTCs marker are selected from the group consisting of an antibody, an antibody derivative, a lectin and an aptamer.

The term "antibody" is used herein in the broadest sense and covers monoclonal antibodies (including full length monoclonal antibodies), polyclonal antibodies, multispecific antibodies (e.g. bispecific antibodies), and antibody fragments so long as they exhibit the desired biological activity. An intact "antibody" includes heteromultimeric glycoproteins comprising at least two heavy (H) chains and two light (L) chains inter-connected by disulphide bonds. Typically, each light chain is linked to a heavy chain by one covalent disulphide bond while the number of disulphide linkages between the heavy chains of different immunoglobulin isotypes varies. Each heavy and light chain also has intra-chain disulphide bridges. Each heavy chain has at one end a heavy chain variable region (abbreviated herein as HCVR or VH) followed by a heavy chain constant region. The heavy chain constant region is comprised of three domains, CH1, CH2, and CH3. Each light chain has a light chain variable region (abbreviated herein as LCVR or VL) and a light chain constant region at its other end. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxyl-terminus in the following order: FR1, CDR1, FR2, CDR2, FR3, CDR3, FR4. The variable regions of the heavy and light chains contain a binding domain that interacts with an antigen. The constant regions are not directly involved in the binding of the antibody to the antigen but exhibit various effector functions such as participation in antibody dependent cell-mediated cytotoxicity (ADCC), phagocytosis via binding to Fey receptor, half- life/clearance rate via neonatal Fc receptor (FcRn) and complement dependent cytotoxicity via the Clq component of the complement cascade.

The term "monoclonal antibody" refers to a preparation of antibody molecules of homogeneous molecular composition i.e. the individual antibodies comprising the population are identical except for possible naturally occurring mutations that may be present in minor amounts. A monoclonal antibody composition displays a single binding specificity and affinity for a particular epitope or antigenic binding site. The monoclonal antibodies are produced by a hybrid cell product of the fusion of a B-cell clone descendent of a single unique parent cell and a tumour plasma cell. Furthermore, in contrast to polyclonal antibody preparations that typically include different antibodies directed against different determinants (epitopes), each monoclonal antibody is directed against a single determinant on the antigen.

The term "polyclonal antibody" refers to a preparation of more than 1 (two or more) different antibodies to an antigen, i.e., antibodies which are a mixture of immunoglobulins, secreted against a specific antigen. Such a preparation includes antibodies binding to a range of different epitopes.

The invention also comprises the use of fragments of the different types of antibodies mentioned above. The term "fragment" when referring to an antibody, means antigen binding fragments of an antibody comprising a partial heavy or light chain variable sequence, which retain capacity to bind the antigen. Examples of fragments include, without being limited to, (i) a Fab fragment, a monovalent fragment consisting of the VL, VH, CL, and CH1 domains; (ii) a F(ab')2 fragment, a bivalent fragment comprising two Fab fragments linked by a disulphide bridge at the hinge region; (iii) a Fd fragment consisting of the VH and CH1 domains; (iv) a Fv fragment consisting of the VL and VH domains of a single arm of an antibody; (v) a dAb fragment (Ward et al, (1989) Nature 341 :544-546), which consists of a VH domain; and (vi) an isolated complementarity determining region (CDR). Fragments can be prepared by recombinant techniques or enzymatic or chemical cleavage of intact antibodies, e.g. papain digestion (see for example, WO94/29348). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); See, e.g., Bird et al. (1988) Science 242:423-426; and Huston et al. (1988) Proc. Natl. Acad. Sci. USA 85:5879-5883). Such single chain antibodies are included by reference to the term "antibody".

An "antibody derivative" means an antibody, as defined above (including an antibody fragment), or Fc domain or region of an antibody, that is modified by covalent attachment of a heterologous molecule or by any other modification not normally associated with the antibody or Fc domain or region. A wide range of antibody derivatives is known in the art (Holliger & Hudson, Nature Biotechnology, 2005, vol 23, 1126-1136; and Wu & Senter, Nature Biotechnology, 2005, vol 23, 1137-1146).

The term "epitope" refers to a protein determinant capable of specific binding to an antibody, or the place where an antibody binds its Ag, or by extension to the peptide presented in an MHC molecule to which a T-cell receptor binds. Epitopes consist of chemically active surface groupings of molecules such as amino acids or sugar side chains and usually have specific three dimensional structural characteristics, as well as specific charge characteristics. Conformational and non-conformational epitopes are distinguished in that the binding to the former but not the latter is lost in the presence of denaturing solvents.

The term "lectin", as used herein, refers to carbohydrate-binding proteins, macromolecules that are highly specific for sugar moieties. Lectins are known to perform recognition on the molecular and cellular level and play numerous roles in biological recognition involving cells, carbohydrates, and proteins.

The term "aptamer", as used herein, refers to single-stranded nucleic acid chains adopting a specific tertiary structure that allows them to bind to molecular targets with high specificity and affinity, comparable to that of monoclonal antibodies, through interactions other than conventional Watson-Crick base pairing. The term "nucleic acid", in the context of the present invention, refers to any type of nucleic acid, such as DNA and RNA, and to variants thereof, such as peptide nucleic acid (PNA), locked nucleic acid (LNA), as well as combinations thereof, modifications thereof, including modified nucleotides, etc. The terms "nucleic acid" and "oligonucleotide" and "polynucleotide" are used interchangeably in the context of the present invention. Nucleic acids can be purified from natural sources, produced using recombinant expression systems and, optionally, purified, chemically synthesized, etc. When appropriate, for example, in the case of chemically synthesized molecules, the nucleic acids can comprise nucleoside analogues such as analogues having chemically modified bases or sugars, modifications of the backbone, etc. A nucleic acid sequence is represented in 5'-3' direction unless indicated otherwise.

In a preferred embodiment of the invention, the first and second probes are antibodies.

The probes of the invention are each labelled with a different label; wherein the labels are independently selected from the group consisting of a fluorescent label, an elastic radiation label, and a Raman label.

In a preferred embodiment, the probes of the invention are labelled with a fluorescent group. The fluorescent group can bind to the probe directly or through a connecting group. Preferably, the fluorescent groups which are used to label the probes must be (i) groups giving a good signal in the absence of background, (ii) stable groups which allow detecting the signal without significant photobleaching, (iii) groups having good solubility in aqueous media to facilitate the labelling process and/or (iv) groups which are not toxic or alter the proteins such that they lose the capacity to bind to their targets. Methods for conjugating fluorescent reagents to polypeptides are well known in the state of the art and have been described, for example, in The Molecular Probes Handbook-A Guide to Fluorescent Probes and Labeling Technologies (ThermoFisher Scientific). Suitable fluorescent compounds for use in the present invention include but are not limited to ethidium bromide, SYBR Green, fluorescein isothiocyanate (FITC), tetramethyl rhodamine isothiol (TRIT), 5-carboxyfluorescein, 6-carboxyfluorescein, fluorescein, HEX (6-carboxy-2', 4, 4', 5' , 7, 7'-hexachlorofluorescein), Oregon Green 488, Oregon Green 500, Oregon Green 514, Joe (6-carboxy-4', 5'-dichloro-2', 1'-dimethoxyfluorescein), 5-carboxy-2', 4', 5', 1'-tetrachlorofluorescein, 5-carboxyrhodamine, rhodamine, tetramethylrhodamine (Tamra), Rox (carboxy-X-rhodamine), R6G (rhodamine 6G), phthalocyanines, azomethines, cyanines (Cy2, Cy3 and Cy5), Peridinin-chlorophyll proteins (PerCP), PerCP/Cy5.5, Texas Red, Princeston Red, BODIPY FL-Br2, BODIPY 530/550, BODIPY T R, BODIPY 558/568, BODIPY 564/570, BODIPY 576/589, BODIPY 581/591, BODIPY TR, BODIPY 630/650, BODIPY 650/665, DABCYL, Eosin, Erythrosine, ethidium bromide, green fluorescent protein (GFP) and the analogues thereof, inorganic fluorescent labels based on semiconductor nanocrystals (Quantum dot), fluorescent labels based on lanthanides such as Eu3+ and Sm3+ and the like. In a preferred embodiment the fluorescent label is FITC. In another embodiment the fluorescent label is Cy5. In yet another embodiment the fluorescent label is PerCP/Cy5.5.

In a preferred embodiment of the invention, the first and second probes are fluorescently labelled.

In a third aspect, the invention also relates to an *in vitro* method for the detection of circulating tumour cells (CTCs) in a fluid sample, wherein the method comprises the steps of:
(a) incubating the fluid sample with a labelling solution wherein the labelling solution comprises a first labelled probe targeting a first CTCs surface marker and at least a second labelled probe targeting a second CTCs surface marker, wherein the first and second probes are fluorescently labelled; or alternatively incubating the fluid sample with a labelling solution wherein the labelling solution comprises a first labelled probe targeting a first CTCs surface marker, wherein the first probe is fluorescently labelled;
(b) removing the excess of any labelled probe not bound to the CTCs, thereby obtaining a labelled fluid sample; and
(c) detecting in a microfluidic device according to the first aspect of the invention a first signal corresponding to the first labelled probe and at least a second signal selected from the group consisting of the at least second labelled probe, autofluorescence, and combinations thereof, in the labelled fluid sample;
wherein the detection of the first signal and the second signal is indicative of the presence of CTCs in the fluid sample; wherein the first and second probes are labelled with a different label; wherein the first and second labels are independently selected from the group consisting of a fluorescent label, an elastic radiation label, and a Raman label; and wherein the first and second probes are independently selected from the group consisting of an antibody, an antibody derivative, a lectin and an aptamer.

All the embodiments and definitions of the second aspect of the invention that are applicable to the third aspect of the invention are included herein.

In a preferred embodiment, the second signal corresponds to the at least second labelled probe. In another preferred embodiment, the second signal corresponds to autofluorescence. In yet another preferred embodiment, the second signal corresponds to the at least second labelled probe and autofluorescence.

The term "autofluorescence", as used herein, refers to the natural emission of light by biological structures within a cell. In the context of the invention, the autofluorescence serves as a marker of cells in the sense that it allows cells to be detected, but does not allow to discern the type of cell.

The expression "CTCs surface marker", as used herein, refers to a protein or receptor present on the surface of the cell membrane of a CTC. It also relates to a carbohydrate or a ganglioside present on the surface of the cell membrane. The CTC surface marker can be a surface marker for a cell of non-haematological lineage, including, without limitation, an epithelial lineage marker, a mesenchymal lineage marker, a stem cell lineage marker and a specific cancer marker.

In a preferred embodiment, the first probe targets a first specific marker for a cell of non-haematological lineage, and the second probe targets a second specific marker for a cell of non-haematological lineage, wherein the specific marker for a cell of non-haematological lineage is selected from the group consisting of a epithelial lineage marker, a mesenchymal lineage marker, a stem cell lineage marker and a specific cancer marker.

In the context of the present invention, the phrase "specific marker for a cell of non-haematological lineage" refers to a marker that is not present in a haematological lineage cell. When the specific marker for a cell of non-haematological lineage is a specific cancer marker, the cancer is not a haematological cancer (i.e. the cancer is a solid tumour).

In the context of the present invention, the term "epithelial lineage marker" refers to a marker that is present in an epithelial lineage cell. In the context of the present invention, the term "mesenchymal lineage marker" refers to a marker that is present in a mesenchymal lineage cell. In the context of the present invention, the term "stem cell lineage marker" refers to a marker that is present in a stem cell lineage cell.

In a preferred embodiment, the mesenchymal lineage marker is selected from the group consisting of 5'-Nucleotidase/ CD73, ALCAM/ CD166, Aminopeptidase N/ CD13, BMPR-IA/ ALK-3, BMPR-IB/ ALK-6, BMPR-II, N-Cadherin, CD44, CD45, CD45.1, CD45.2, CD90/ Thy1, CD117/ c-kit, CD45RO, Endoglin/ CD105, Fibronectin, Fibronectin/ Anastellin, HLA Class I, ICAM-1/ CD54, Integrin alpha 1/ CD49a, Integrin alpha 5/ CD49e, Integrin alpha V/ CD51, Integrin beta 1/ CD29, NCAM-1/ CD56, NGF R/ TNFRSF16, Nucleostemin, PDGF R alpha, Sca-1/ Ly6, SSEA-4, STRO-1, SUSD2, TfR (Transferrin R), VCAM-1/ CD106 and Vimentin.

In a preferred embodiment, the stem cell lineage marker is selected from the group consisting of Oct-4, SSEAs, CD34, CD133, ABCG2, Sca-1, STRO-1, Nestin, PSA-NCAM and p75 neurotrophin receptor (NTR).

In a more preferred embodiment, the first probe targets a specific marker for a cell of epithelial lineage, and/or the second probe targets a specific cancer marker.

In a preferred embodiment, the specific marker for a cell of epithelial lineage is selected from the group consisting of EpCAM, CDH1, cytokeratin, MAL, TGFβ-R1, TNF-R1, Ki1, CEA, PSA, CD31, FGFR2, FGFR3, CD44, PSGL1, PD-L1, I-CAM, V-CAM, alpha-V beta-3, alpha-V beta-5, alpha-V beta-6, alpha-VI beta-4, alpha-II beta-1, alpha-R beta-1, alpha-V beta-1, WNT, CD24, Sialyl Lewis A, Sialyl Lewis X, T antigen, Tn antigen, Sialyl Tn, GD2, GD3, GD1a, MUC1, MUC3, MUC2, MUC4, MUC5AC and MUC5B; and/or wherein the specific cancer marker is selected from a breast cancer marker, a prostate cancer marker, a lung cancer marker, and a colon cancer marker;

The term "EpCAM", also known as KS1/4, TROP1, TACST-1, MK-1, M1S2, ESA, M4S1, HNPCC8, 323/A3, CD326, DIAR5, Ep-CAM, Ly74, EGP40, 17-1A, KSA, EGP314, TACSTD1, MIC18, MOC31, HEA125, CO-17A, MH99, EGP-2, GA733-2, EGP34, as used herein, refers to the epithelial cell adhesion molecule. The human gene that codifies said protein is shown in the Ensembl database under accession number ENSG00000119888. In a preferred embodiment the specific marker for a cell of epithelial lineage is EpCAM.

The term "CDH1", also known as cadherin 1, CDHE, Arc-1, uvomorulin, ECAD, UVO, LCAM, CD324, as used herein, refers to E-cadherin. The human gene that codifies said protein is shown in the Ensembl database under accession number ENSG00000039068.

The term "cytokeratin", as used herein, refers to acidic type I cytokeratins and basic or neutral type II cytokeratins. Cytokeratins are usually found in pairs comprising a type I cytokeratin and a type II cytokeratin. Basic or neutral cytokeratins include CK1, CK2, CK3, CK4, CK5, CK6, CK7, and CK8. Acidic cytokeratins are CK9, CK10, CK12, CK13, CK14, CK16, CK17, CK18, CK19 and CK20.

The term "MAL", as used herein, refers to the myelin and lymphocyte protein. The human gene that codifies said protein is shown in the Ensembl database under accession number ENSG00000172005.

The term "TGFβ-R1", also known as TGFBR1, tbetaR-I, LDS2A, MSSE, ACVRLK4, AAT5, ALK-5, SKR4, LDS1, ESS1, TGFR-1, ALK5, LDS1A, as used herein, refers to the transforming growth factor beta receptor 1. The human gene that codifies said protein is shown in the Ensembl database under accession number ENSG00000106799.

The term "TNF-R1", also known as TNFR1, TNFR60, TNFAR, TNF-R55, TBP1, TNFR55, TNF-R-I, p60, CD120a, FPF, p55-R, TNF-R, p55, as used herein, refers to the TNF receptor superfamily member 1A. The human gene that codifies said protein is shown in the Ensembl database under accession number ENSG00000067182.

The term "Ki1", also known as TNFRSF8, D1S166E, KI-1, Ki-1, CD30, as used herein, refers to the TNF receptor superfamily member 8. The human gene that codifies said protein is shown in the Ensembl database under accession number ENSG00000120949.

The term "CEA", also known as CEACAM1, BGP1, BGPI, CD66a, BGP, as used herein, refers to the carcinoembryonic antigen related cell adhesion molecule 1. The human gene that codifies said protein is shown in the Ensembl database under accession number ENSG00000079385.

The term "PSA", also known as KLK3, KLK2A1, hK3, APS, as used herein, refers to kallikrein related peptidase 3. The human gene that codifies said protein is shown in the Ensembl database under accession number ENSG00000142515.

The term "CD31", also known as PECAM1, endoCAM, CD31/EndoCAM, PECAM-1, PECA1, GPIIA, as used herein, refers to the platelet and endothelial cell adhesion molecule 1. The human gene that codifies said protein is shown in the Ensembl database under accession number ENSG00000261371.

The term "FGFR2", also known as CD332, TK14, JWS, ECT1, BEK, BBDS, KGFR, TK25, CFD1, K-SAM, BFR-1, CEK3, KSAM, as used herein, refers to the fibroblast growth factor receptor 2. The human gene that codifies said protein is shown in the Ensembl database under accession number ENSG00000066468.

The term "FGFR3", also known as HSFGFR3EX, JTK4, CEK2, CD333, ACH, as used herein, refers to the fibroblast growth factor receptor 3. The human gene that codifies said protein is shown in the Ensembl database under accession number ENSG00000068078.

The term "CD44", also known as LHR, MDU2, HUTCH-I, CDW44, CSPG8, Pgp1, IN, MDU3, MIC4, MC56, ECMR-III, HCELL, CD44R, CD44 molecule (Indian blood group), HCAM, as used herein, refers to the homing cell adhesion molecule. The human gene that codifies said protein is shown in the Ensembl database under accession number ENSG00000026508.

The term "PSGL1", also known as SELPLG, PSGL-1, CD162, CLA, as used herein, refers to the selectin P ligand. The human gene that codifies said protein is shown in the Ensembl database under accession number ENSG00000110876.

The term "PD-L1", also known as CD274, PDCD1L1, B7-H, B7-H1, PDCD1LG1, PDL1, B7H1, as used herein, refers to the programmed death-ligand 1. The human gene that codifies said protein is shown in the Ensembl database under accession number ENSG00000120217.

The term "I-CAM", also known as CD54, P3.58, BB2, as used herein, refers to the intercellular adhesion molecule 1. The human gene that codifies said protein is shown in the Ensembl database under accession number ENSG00000090339.

The term "V-CAM", also known as V-CAM1, CD106, L1CAM, INCAM-100, as used herein, refers to the vascular cell adhesion molecule 1. The human gene that codifies said protein is shown in the Ensembl database under accession number ENSG00000162692.

The terms "alpha-V beta-3", "alpha-V beta-5", "alpha-V beta-6", "alpha-VI beta-4", "alpha-II beta-1", "alpha-R beta-1", and "alpha-V beta-1", as used herein, refer to the glycoprotein transmembrane receptors integrins. Integrins are obligate heterodimers and they are formed of two subunits: α (alpha) and β (beta). Integrins in mammals have eighteen α and eight β subunits.

The term "WNT1", also known as BMND16, INT1, 0115, as used herein, refers to the Wnt family member 1. The human gene that codifies said protein is shown in the Ensembl database under accession number ENSG00000125084.

The term "CD24", also known as CD24A, as used herein refers to the CD24 molecule. The human gene that codifies said protein is shown in the Ensembl database under accession number ENSG00000272398.

The term "Sialyl-Lewis A", also known as sialyl Le^{A} and SLe^{A}, carbohydrate antigen 19-9, cancer antigen 19-9 (CA 19-9), as used herein, refers to a tetrasaccharide carbohydrate that is usually attached to Lewis A O-glycans on the surface of cells. Occurrence of the Sialyl-Lewis A antigen, detected with the CA19-9 antibody, is highly correlated advanced epithelial cancers, such as stage III and stage IV colorectal cancers.

The term "Sialyl-Lewis X", also known as sialyl Le^{x} and SLe^{x}, as used herein, refers to a tetrasaccharide carbohydrate that is usually attached to O-glycans on the surface of cells.

The term "T antigen", also known as the Thomsen-Friedenreich antigen or TF antigen, as used herein, refers to a disaccharide antigen formed by addition of galactose (Gal(b1-3)GalNAc) to the Tn antigen.

The term "Tn antigen", as used herein, refers to the "oligosaccharide" (monosaccharide) structure N-acetylgalactosamine (GalNAc) linked to serine or threonine by a glycosidic bond, i.e. as an O-glycan. The Tn antigen is not usually found on healthy cell surfaces, but may be found on cancer cells.

The term "sialyl Tn antigen", also known as STn antigen, as used herein, refers to a disaccharide antigen formed by addition of sialic acid (Neu5Ac(a2-6)GalNAc) to the Tn antigen.

The term "GD2", as used herein, refers to the ganglioside bDGalpNAc(1-4)[aNeu5Ac(2-8)aNeu5Ac(2-3)]bDGalp(1-4)bDGlcp(1-1)Cer.

The term "GD3", as used herein, refers to the ganglioside aNeu5Ac(2-8)aNeu5Ac(2-3)bDGalp(1-4)bDGlcp(1-1)Cer.

The term "GD1a", as used herein, refers to the ganglioside aNeu5Ac(2-3)bDGalp(1-3)bDGalNAc(1-4)[aNeu5Ac(2-3)]bDGalp(1-4)bDGlcp(1-1)Cer.

The term "MUC1", also known as MUC-1/X, PEMT, ADMCKD1, MCD, MUC-1/SEC, MCKD, PUM, ADMCKD, MCKD1, KL-6, CA 15-3, MUC1/ZD, MUC-1, PEM, EMA, H23AG, MAM6, CD227, as used herein, refers to mucin 1. The human gene that codifies said protein is shown in the Ensembl database under accession number ENSG00000185499.

The term "MUC2", as used herein, refers to mucin 2. The human gene that codifies said protein is shown in the Ensembl database under accession number ENSG00000198788.

The term "MUC3", also known as MUC-3A, as used herein, refers to mucin 3A. The human gene that codifies said protein is shown in the Ensembl database under accession number ENSG00000169894.

The term "MUC4", also known as HSA276359, MUC-4, ASGP, as used herein, refers to mucin 4. The human gene that codifies said protein is shown in the Ensembl database under accession number ENSG00000145113.

The term "MUC5AC", also known as mucin, MUC5, leB, TBM, as used herein, refers to mucin 5AC. The human gene that codifies said protein is shown in the Ensembl database under accession number ENSG00000215182.

The term "MUC5B", also known as mucin, MUC5, MUC-5B, MG1, MUC9, as used herein, refers to mucin 5B. The human gene that codifies said protein is shown in the Ensembl database under accession number ENSG00000117983.

The term "cancer marker", in the context of the present invention, refers to protein, lipid, and/or carbohydrate components of the cell membrane which are found exclusively or at higher than normal levels in the cell membrane of cancer cells.

The expression "specific cancer marker" relates to cancer markers which are exclusively or predominantly found in the cell membrane of cancer cells of a specific type of cancer.

The term "cancer" is referred to a disease characterized by uncontrolled cell division (or by an increase of survival or apoptosis resistance) and by the ability of said cells to invade other neighbouring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. Biological processes known to be related to cancer include angiogenesis, immune cell infiltration, cell migration and metastasis. The term cancer includes, without limitation, lung cancer, sarcoma, malignant melanoma, pleural mesothelioma, bladder carcinoma, prostate cancer, pancreas carcinoma, gastric carcinoma, ovarian cancer, hepatoma, breast cancer, colorectal cancer, kidney cancer, oesophageal cancer, suprarenal cancer, parotid gland cancer, head and neck carcinoma, cervix cancer, endometrial cancer, liver cancer, mesothelioma, multiple myeloma, leukaemia, and lymphoma. In a particular embodiment of the invention, the cancer is breast cancer. The term "breast cancer" relates to any malignant proliferative disorder of breast cells, most commonly from the inner lining of milk ducts or the lobules that supply the ducts with milk. Cancers originating from ducts are known as ductal carcinomas, while those originating from lobules are known as lobular carcinomas.

The specific cancer marker is selected from a breast cancer marker, a prostate cancer marker, a lung cancer marker and a colon cancer marker.

In a preferred embodiment the specific cancer marker is a breast cancer marker. The specific breast cancer marker can be, without limitation, estrogen receptor (ER), progesterone receptor (PR), human epidermal growth factor receptor 2 (HER2), cancer antigen 15-3 (CA 15-3), cancer antigen 27.29 (CA 27-29) or carcinoembryonic antigen (CEA). Preferably, the breast cancer marker is HER2.

The term "HER2", also known as ERBB2, MLN19, CD340, HER-2, NGL, NEU, TKR1, MLN 19, HER-2/neu, as used herein, refers to the erb-b2 receptor tyrosine kinase 2. The human gene that codifies said protein is shown in the Ensembl database under accession number ENSG00000141736.

The term "ER", also known as ESR1, NR3A1, ESR, Era, ESRA, ESTRR, as used herein, refers to the estrogen receptor 1. The human gene that codifies said protein is shown in the Ensembl database under accession number ENSG00000091831.

The term "PR", also known as PGR, NR3C3, as used herein, refers to the progesterone receptor. The human gene that codifies said protein is shown in the Ensembl database under accession number ENSG00000082175.

The specific prostate cancer marker can be, without limitation, prostatic acid phosphatase (PAP), human kallikrein 3 (PSA), human kallikrein 2 (hK2), insulin-like growth factor-1 receptor (IGF1R) and insulin-like growth factor binding proteins (IGFPBs), transforming growth factor-β1 (TGF-β1), interleukin-6 (IL-6), prostate-specific membrane antigen (PSMA) or fatty acid synthase (FAS).

The term "PAP", also known as ACPP, ACP-3, 5'-NT, ACP3, as used herein, refers to the prostatic acid phosphatase. The human gene that codifies said protein is shown in the Ensembl database under accession number ENSG00000014257.

The term "hK2", also known as KLK2, KLK2A2, hGK-1, as used herein, refers to the human kallikrein 2. The human gene that codifies said protein is shown in the Ensembl database under accession number ENSG00000167751.

The term "IGF1R", also known as MGC18216, IGFR, IGFIR, JTK13, CD221, as used herein, refers to the insulin like growth factor 1 receptor. The human gene that codifies said protein is shown in the Ensembl database under accession number ENSG00000140443.

The term "PSMA", also known as FOLH, FOLH1, GCP2, PSMAL, FOLH2, PSM, NAALAdase, mGCP, FOLHP, GCPIII, GCPII, PSMA-LIKE, FGCP, NAALAD1, GCP3, as used herein, refers to the prostate-specific membrane antigen, or folate hydrolase 1. The human gene that codifies said protein is shown in the Ensembl database under accession number ENSG00000086205.

The term "FAS", also known as FASN, OA-519, SDR27X1, as used herein, refers to the fatty acid synthase. The human gene that codifies said protein is shown in the Ensembl database under accession number ENSG00000169710.

The specific lung cancer marker can be, without limitation, neuron specific enolase (NSE), epidermal growth factor receptor (EGFR), delta like canonical Notch ligand 3 (DLL3), carcinoembryonic antigen (CEA), keratin 19 (CK19), squamous cell carcinoma antigen (SCC) or cancer antigen 125 (CA 125).

The term "NSE", also known as ENO2, HEL-S-279, as used herein, refers to the neuron specific enolase, or enolase 2. The human gene that codifies said protein is shown in the Ensembl database under accession number ENSG00000111674.

The term "EGFR", also known as ERBB1, PIG61, ERBB, mENA, NISBD2, HER1, as used herein, refers to the epidermal growth factor receptor. The human gene that codifies said protein is shown in the Ensembl database under accession number ENSG00000146648.

The term "DLL3", also known as SCDO1, as used herein, refers to the delta like canonical Notch ligand 3. The human gene that codifies said protein is shown in the Ensembl database under accession number ENSG00000090932.

The term "CK19", also known as KRT19, K19, MGC15366, K1CS, CYFRA 21-1, as used herein, refers to keratin 19. The human gene that codifies said protein is shown in the Ensembl database under accession number ENSG00000171345.

The term "SCC", also known as SERPINB3, SCCA-PD, HsT1196, SCCA, SCCA-1, SCCA1, T4-A, SSCA1, as used herein, refers to squamous cell carcinoma antigen, or serpin family B member 3. The human gene that codifies said protein is shown in the Ensembl database under accession number ENSG00000057149.

The specific colon cancer marker can be, without limitation, cancer antigen 19-9 (CA 19-9) or carcinoembryonic antigen (CEA).

In a preferred embodiment, the first probe targets the epithelial cell adhesion molecule EpCAM, and the second probe targets the receptor tyrosine-protein kinase HER2. In a preferred embodiment, the tumour is a breast tumour, more preferably a HER2 positive breast tumour, and preferably a metastatic breast tumour.

The conditions of the incubation of the fluid sample with the labelling solution are well known by the person skilled in the art.

Step (b) of the second and third aspects of the invention involves removing the excess of any labelled probe not bound to the CTCs, thereby obtaining a labelled fluid sample. Said removal may be done, without limitation, by changing the labelling solution; or by washing with a solution such as a buffer, a cell culture medium or water; or by the use of a microfluidic device; or by centrifugation.

The final step of the second and third aspects of the invention involves the use of a device according to the first aspect of the invention.

In a preferred embodiment, the cells detected are simultaneously labelled with both the first and second labelled probe.

### An in vitro method for diagnosing a tumour and/or metastasis in a subject

In a fourth aspect, the invention relates to an *in vitro* method for diagnosing a tumour and/or metastasis in a subject, wherein the method comprises the step of detecting the presence of CTCs in a fluid sample from said subject by a method according to the second or third aspects of the invention; wherein the detection of the presence of CTCs in the fluid sample is indicative that the subject suffers from a tumour and/or metastasis.

The term "diagnosing", as used herein, refers to assessing the probability according to which a subject is suffering from a disease. As will be understood by those skilled in the art, such assessment, although preferred to be, may usually not be correct for 100% of the subjects to be diagnosed. The term, however, requires that a statistically significant portion of subjects can be identified as suffering from the disease or as having a predisposition therefore. Whether a portion is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90%, at least 95%. The p-values are, preferably, 0.2, 0.1, 0.05.

The term "tumour", as used herein, refers to an abnormal mass of tissue which may be solid or fluid-filled. The term tumour includes pre-malignant lesions such as dysplasia, metaplasia, aplasia and encapsulated tumours, and malignant tumours. In a preferred embodiment the tumour is a malignant tumour. However, in the context of this invention, the term "tumour" does not include benign tumours.

The term "metastasis" is understood as the distance propagation, fundamentally but not limited by the lymphatic, peritoneal or blood stream, of the cancer causing cells, and the growth of new tumours in the destination sites of said metastasis.

"Subject" in the present invention is understood as any animal classified as mammal and includes but is not limited to domestic and farm animals, primates and humans, for example human beings, non-human primates, cows, horses, pigs, sheep, goats, dogs, cats or rodents. Preferably, the subject is a female or male human being of any race or age. In the context of the present invention, the subject is a subject who potentially suffers from a tumour or has been previously diagnosed with cancer.

In a preferred embodiment, the tumour is a breast tumour, more preferably a HER2 positive breast tumour, and preferably a metastatic breast tumour.

The terms "fluid sample", "CTC", and "cancer" have been defined elsewhere herein and apply equally to the fourth aspect of the invention.

### An in vitro method for quantifying circulating tumour cells (CTCs)

In a fifth aspect, the invention relates to an *in vitro* method for quantifying circulating tumour cells (CTCs) in a fluid sample, wherein the method comprises the steps of:
(a) detecting the presence of CTCs in a fluid sample from said subject by a method according the second or third aspects of the invention; and
(b) quantifying the number of CTCs present in the fluid sample from said subject.

The number of CTCs present in the fluid sample can be an absolute value; a relative value; a value that has an upper and/or lower limit; a range of values; an average value; a median value, a mean value, or a value as compared to a particular control or baseline value.

In a preferred embodiment, the tumour is a breast tumour, more preferably a HER2 positive breast tumour, and preferably a metastatic breast tumour.

The terms "fluid sample", "tumour", "CTC", and "cancer" have been defined elsewhere herein and apply equally to the fifth aspect of the invention.

### An in vitro method for determining the prognosis of a subject suffering from a tumour and/or metastasis

In a sixth aspect, the invention relates to an *in vitro* method for determining the prognosis of a subject suffering from a tumour and/or metastasis, wherein the method comprises the steps of:
(a) detecting the presence of CTCs in a fluid sample from said subject by a method according to the second or third aspect of the invention;
(b) quantifying the number of CTCs present in the fluid sample from said subject; and
(c) comparing the number of CTCs present in the fluid sample from said subject with the number of CTCs present in a reference sample;
wherein an increase in the number of CTCs in the fluid sample with respect to the number of CTCs in the reference sample is indicative that the subject has a poor prognosis, or wherein a decrease in the number of CTCs in the fluid sample with respect to the number of CTCs in the reference sample is indicative that the subject has a good prognosis.

As used in the present invention, the expression "determining the prognosis", relates to the determination of one or several parameters indicating the possible outcome of the disease in a patient diagnosed with cancer and/or metastasis. Parameters suitable for determining the evolution of a subject diagnosed with cancer and/or metastasis are selected from the group consisting of risk of relapse, disease-free survival and/or overall survival of the subject. As used herein, the expression "risk of relapse" is understood as the probability of a subject developing cancer or metastasis after a disease-free period; "disease-free survival" is understood as the time period after the treatment in which the cancer is not found; and "overall survival of the subject" is understood as the percentage of subjects who survive, from the time of the diagnosis or treatment, after a defined time period.

Any parameter which is widely accepted for determining the outcome of a patient can be used in the present invention including, without limitation:
- overall survival rate, as used herewith, relates to the percentage of subjects in a study or treatment group who are alive for a certain period of time after they were diagnosed with or treated for a disease, such as cancer.
- disease-specific survival rate which is defined as the percentage of subjects in a study or treatment group who have not died from a specific disease in a defined period of time.
- disease-free survival (DFS), as used herewith, is understood as the length of time after treatment for a disease during which a subject survives with no sign of the disease.
- objective response which, as used in the present invention, describes the proportion of treated subjects in whom a complete or partial response is observed.
- tumour control which, as used in the present invention, relates to the proportion of treated subjects in whom complete response, partial response, minor response or stable disease ≥ 6 months is observed.
- progression free survival which, as used herein, is defined as the time from start of treatment to the first measurement of cancer growth.
- time to progression (TTP), as used herein, relates to the time since a disease is treated until the disease starts to get worse.
- six-month progression free survival or "PFS6" rate which, as used herein, relates to the percentage of subjects who are free of progression in the first six months after the initiation of the therapy and
- median survival which, as used herein, relates to the time at which half of the subjects enrolled in the study are still alive.

The term "reference sample", as used in the context of the sixth aspect of the invention, refers to a laboratory value used as a reference for values/data obtained by laboratory examinations of subjects or samples collected from subjects. The reference value or reference level can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject being tested, but at an earlier point in time or from a non-cancerous tissue. The reference value can be based on a large number of samples, such as from population of subjects of the chronological age matched group, or based on a pool of samples including or excluding the sample to be tested. Various considerations are taken into account when determining the reference value. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

In a preferred embodiment, the reference value is the average number of CTCs present in fluid samples from a pool of patients. This pool will include patients with good prognosis and patients with bad prognosis and therefore, the average number of CTCs present in fluid samples would be an average value of the values found in the different types of patients. In another embodiment, the reference value is the average number of CTCs present in fluid samples from a patient or patients identified as patients having a good prognosis. In another embodiment, the reference value is the average number of CTCs present in fluid samples from a patient or patients identified as patients having a bad prognosis. In case that the reference value is the average number of CTCs present in fluid samples from patients having a good prognosis, then patients can be identified as having good prognosis if the number of CTCs present in a fluid sample is lower than the reference value. In case that the reference value is the average number of CTCs present in fluid samples from patients having a poor prognosis, then patients can be identified as having poor prognosis if the number of CTCs present in a fluid sample is higher than the reference value. The sample collection from which the reference sample is derived will preferably be formed by subjects suffering from the same type of cancer as the patient object of the study.

In a preferred embodiment, the reference sample is a first sample obtained from the same subject at an earlier time of point of the disease.

In the context of the present invention, a "decrease" in the number of CTCs with respect to the reference sample is understood as a variation in the number of CTCs under the reference sample of at least 0.9 times, 0.75 times, 0.5 times, 0.2 times, 0.1 times, 0.05 times, 0.025 times, 0.02 times, 0.01 times, 0.005 times or even less compared to the reference sample.

In the context of the present invention, an "increase" in the number of CTCs with respect to the reference sample is understood as a variation in the number of CTCs above the reference sample of at least 1.1 times, 1.5 times, 2 times, 5 times, 10 times, 20 times, 30 times, 40 times, 50 times, 60 times, 70 times, 80 times, 90 times, 100 times or even more times as compared to the reference sample.

In a preferred embodiment, the tumour is a breast tumour, more preferably a HER2 positive breast tumour, and preferably a metastatic breast tumour.

The terms "cancer", "tumour", "metastasis", "subject", "CTC", and "fluid sample" have been previously defined and apply equally to the sixth aspect of the invention.

### An in vitro method for monitoring the progression of a tumour and/or metastasis in a subject

In another aspect, the invention relates to an *in vitro* method for monitoring the progression of a tumour and/or metastasis in a subject, wherein the method comprises the steps of:
(a) detecting the presence of CTCs in a fluid sample from said subject by a method according to the second or third aspect of the invention;
(b) quantifying the number of CTCs present in the fluid sample from said subject; and
(c) comparing the number of CTCs present in the fluid sample from said subject with the number of CTCs present in a reference sample obtained from the same subject at an earlier time point of the disease;
wherein an increase in the number of CTCs in the fluid sample with respect to the number of CTCs in the reference sample is indicative of tumour progression, or wherein a decrease in the number of CTCs in the fluid sample with respect to the number of CTCs in the reference sample is indicative of tumour regression or remission.

As used in the present invention, the expression "monitoring the progression", relates to the evaluation of the progression of cancer in a selected subject previously diagnosed as suffering from cancer and/or metastasis. Consequently, if the cancer and/or metastasis is in progression, the subject may be selected to receive an appropriate treatment. The progression of the cancer after said treatment can be easily followed according to the teachings of this invention.

The term "reference sample", as used in the context of this aspect of the invention, refers to a first sample obtained from the same subject at an earlier time of point of the disease. A second sample is taken from the same subject having cancer from which the first sample is derived, at a second period of time, i.e., at any time after the first period of time, e.g., one day, one week, one month, two months, three months, 1 year, 2 years, or more after the first sample. In a particular embodiment, the first sample is taken prior to the subject receiving treatment, e.g. chemotherapy, radiation therapy, or surgery, and the second sample is taken after treatment. In another particular embodiment, the first sample is taken after the subject has started/received treatment, e.g. chemotherapy, radiation therapy, or surgery, and the second sample is taken later, at different time periods during a course of treatment.

In the context of the present invention, a "decrease" in the number of CTCs with respect to the reference sample is understood as a variation in the number of CTCs under the reference sample of at least 0.9 times, 0.75 times, 0.5 times, 0.2 times, 0.1 times, 0.05 times, 0.025 times, 0.02 times, 0.01 times, 0.005 times or even less compared to the reference sample.

In the context of the present invention, an "increase" in the number of CTCs with respect to the reference sample is understood as a variation in the number of CTCs above the reference sample of at least 1.1 times, 1.5 times, 2 times, 5 times, 10 times, 20 times, 30 times, 40 times, 50 times, 60 times, 70 times, 80 times, 90 times, 100 times or even more times as compared to the reference sample.

Thus, a significant decrease in the number of CTCs with respect to the reference sample is indicative that the cancer is not in progression (i.e. the cancer is responding to treatment). On the contrary, if a significant increase in the number of CTCs with respect to the reference sample is detected, this is indicative that the cancer is in progression (i.e. the cancer is not responding to treatment). Thus, the therapy administered to the subject under study should be changed and a new therapy should be designed to treat cancer.

In a preferred embodiment, the tumour is a breast tumour, more preferably a HER2 positive breast tumour, and preferably a metastatic breast tumour.

The terms "cancer", "tumour", "metastasis", "subject", "CTC", and "fluid sample" have been previously defined and apply equally to the present aspect of the invention.

### An in vitro method for monitoring the response to a therapy in a subject suffering from cancer and/or metastasis

In another aspect, the invention relates to an *in vitro* method for monitoring the response to a therapy in a subject suffering from cancer and/or metastasis, wherein the method comprises the steps of:
(a) detecting the presence of CTCs in a fluid sample from said subject obtained after the therapy is administered, said detection carried out by a method according to the second or third aspect of the invention;
(b) quantifying the number of CTCs present in the fluid sample from said subject obtained after the therapy is administered; and
(c) comparing the number of CTCs present in the fluid sample from said subject with the number of CTCs present in a reference sample obtained from the same subject before the therapy is administered;
wherein an increase in the number of CTCs in the fluid sample with respect to the number of CTCs in the reference sample is indicative that the subject does not respond to therapy, or wherein a decrease or a lack of change in the number of CTCs in the fluid sample with respect to the number of CTCs in the reference sample is indicative that the subject responds to therapy.

The expression "monitoring the response to a therapy", as used herein, refers to the follow-up of a disease during a treatment to determine if said treatment is effective or not against said disease.

As used herein, the term "therapy" or "treatment" collectively refers to the means of any class, hygienic means, pharmacological means, surgical means or physical means, the purpose of which is to prevent and/or cure or relieve a disease or pathology or its symptoms. In cancer therapy, a variety of treatments can be used in an attempt to eliminate or contain the cancer. Treatment for cancer may involve active surveillance, hormonal therapy, radiation therapy, chemotherapy or some combination. In a specific case, said treatment is a pharmacological treatment, i.e., a treatment comprising the administration of a drug to a subject to prevent, relieve and/or cure a disease, e.g., cancer, or to relieve, reduce or eliminate one or more symptoms associated with said disease. Best decision is made depending on the stage of the disease.

Briefly, depending on several factors (e.g., age of the subject, as well as the size, location and cancer phase), (i) cytotoxic/cytostatic treatments, such as chemotherapy, which uses medicinal products against cancer to destroy the cancerous cells upon making the medicinal products circulate through the body through the blood vessels; radiotherapy, which uses high energy radiations to kill the cancer cells, and anti-tumour agents; and/or (ii) immunotherapy, wherein the administered compound stimulates, enhances or strengthens the natural function of the immune system against cancer to recognise and eliminate the cancerous cells from the body, can be used. Thus, the method of the invention allows determining the response of the subject having cancer to any treatment, particularly, to any cytotoxic and/or cytostatic treatment and, more specifically, to a treatment by means of chemotherapy, radiotherapy, anti-tumour agents or combinations thereof.

Suitable chemotherapy agents include but are not limited to alkylating agents [e.g., Cisplatin, Carboplatin, Oxaliplatin, BBR3464, Chlorambucil, Chlormethine, Cyclophosphamides, Ifosmade, Melphalan, Carmustine, Fotemustine, Lomustine, Streptozocin, Busulfan, Dacarbazine, Mechlorethamine, Procarbazine, Temozolomide, ThioTPA, Uramustine, etc.]; anti-metabolites [e.g., purine (azathioprine, mercaptopurine), pyrimidine (Capecitabine, Cytarabine, Fluorouracil, Gemcitabine), folic acid (Methotrexate, Pemetrexed, Raltitrexed), etc.]; vinca alkaloids [e.g., Vincristine, Vinblastine, Vinorelbine, Vindesine, etc.]; a taxane [e.g., paclitaxel, docetaxel, BMS-247550, etc.]; an anthracycline [e.g., Daunorubicin, Doxorubicin, Epirubicin, Idarubicin, Mitoxantrone, Valrubicin, Bleomycin, Hydroxyurea, Mitomycin, etc.]; a topoisomerase inhibitor [e.g., Topotecan, Irinotecan Etoposide, Teniposide, etc.]; a monoclonal antibody [e.g., Alemtuzumab, Bevacizumab, Cetuximab, Gemtuzumab, Panitumumab, Rituximab, Trastuzumab, etc.]); a photosensitizer [e.g., Aminolevulinic acid, Methyl aminolevulinate, Porfimer sodium, Verteporfin, etc.]; a tyrosine kinase inhibitor [e.g., Gleevec(TM)]; an epidermal growth factor receptor inhibitor [e.g., Iressa(TM), erlotinib (Tarceva(TM)), gefitinib, etc.]; an FPTase inhibitor [e.g., FTIs (R1 15777, SCH66336, L- 778,123), etc.]; a KDR inhibitor [e.g., SU6668, PTK787, etc.]; a proteosome inhibitor [e.g., PS341, etc.]; a TS/DNA synthesis inhibitor [e.g., ZD9331, Raltirexed (ZD 1694, Tomudex), ZD9331, 5-FU, etc.]; an S-adenosyl-methionine decarboxylase inhibitor [e.g., SAM468A, etc.]; a DNA methylating agent [e.g., TMZ, etc.]; a DNA binding agent [e.g., PZA, etc.]; an agent which binds and inactivates O6-alkylguanine AGT [e.g., BG]; a c-ra/-1 antisense oligo-deoxynucleotide [e.g., ISIS-5132 (CGP- 69846A)]; tumour immunotherapy; a steroidal and/or non-steroidal anti-inflammatory agent [e.g., corticosteroids, COX-2 inhibitors]; or other agents such as Alitretinoin, Altretamine, Amsacrine, Anagrelide, Arsenic trioxide, Asparaginase, Bexarotene, Bortezomib, Celecoxib, Dasatinib, Denileukin Diftitox, Estramustine, Hydroxycarbamide, Imatinib, Pentostatin, Masoprocol, Mitotane, Pegaspargase, and Tretinoin. Suitable chemotherapy agents are described with more detail in the literature, such as in The Merck Index in CD-ROM, 13th edition.

This method allows for the evaluation of a particular treatment for a selected subject previously diagnosed with cancer and/or metastasis. Consequently, if the therapy is not efficacious for treating cancer in said subject, then said therapy should be changed and a new therapy should be designed to treat cancer in said subject. The course of the new treatment can be easily followed according to this method.

In a preferred embodiment, the tumour is a breast tumour, more preferably a HER2 positive breast tumour, and preferably a metastatic breast tumour.

The terms "cancer", "tumour", "metastasis", "subject", "CTC", "fluid sample", "decrease" and "increase" have been previously defined in the context of the previous methods of the invention.

### A kit

In another aspect, the invention relates to a kit comprising:
(a) a microfluidic device according to the first aspect of the invention, and
(b) a first labelled probe targeting a first CTCs surface marker and, optionally, at least a second labelled probe targeting a second CTCs surface marker
wherein the first and second probes are labelled with a different label and wherein the first and second labels are independently selected from the group consisting of a fluorescent label, an elastic radiation label, and a Raman label; and wherein the first and second probes are independently selected from the group consisting of an antibody, an antibody derivative, a lectin and an aptamer.

In the context of the present invention, "kit" or "assay device" is understood as a product or device containing the different reagents necessary for carrying out the methods of the invention packed so as to allow their transport and storage. Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet addresses that provide said instructions.

The expression "a first labelled probe targeting a first CTCs surface marker and at least a second labelled probe targeting a second CTCs surface marker" means reagents that allow quantifying the number of CTCs present in the fluid sample according to the methods of the invention. Thus, the reagents include compounds that bind specifically with the proteins encoded by the CTCs marker genes and preferably include antibodies and antibody derivatives, although they can be specific aptamers. It also includes reagents that bind specifically to carbohydrates or gangliosides present on the CTC cell membrane surface, such as lectins.

In a preferred embodiment, the reagents adequate for quantifying the number of CTCs present in the fluid sample comprise at least 55%, at least 60%, at least 65%, at least 70%, at least 75%, at least 80%, at least 90%, at least 95%, at least 96%, at least 97%, at least 98% or at least 99% of the total amount of reagents forming the kit.

All the embodiments disclosed in the context of the previous aspects of the invention are also applicable to the kit of the invention.

Thus, the invention refers to the following aspects
1. A microfluidic device for the detection of circulating tumour cells (CTCs) in a fluid sample comprising:
   (a) a cell focusing element selected from a hydrodynamic focusing element and an inertial focusing element, wherein
      the hydrodynamic focusing element comprises at least three inlets, a first central inlet for the entrance of a labelled fluid sample, and a second and third inlets for the entrance of a focusing fluid for flow focusing of the labelled fluid sample in a first plane, wherein the second and third inlets are situated at opposite sides of the first central inlet, and optionally, a fourth and fifth inlets for the entrance of a focusing fluid for flow focusing of the labelled fluid sample in a second plane perpendicular to the first plane, wherein the fourth and fifth inlets are situated at opposite sides of the first central inlet, all the inlets converging into a single microfluidic channel; and wherein
      the inertial focusing element comprises a functional microchannel selected from the group consisting of a straight channel, a straight channel with pillar arrays, a straight channel with expansion-contraction arrays, a spiral channel, a serpentine channel, and combinations thereof;
   (b) a single microfluidic channel comprising at least two interrogation regions, wherein each interrogation region comprises at least one waveguide for labelled fluid sample excitation, and at least one waveguide for signal detection, wherein the at least one waveguide for signal detection is not placed at a 180 degree angle with respect to the at least one waveguide for sample excitation, wherein the microfluidic channel optionally comprises lenses and/or mirrors for ensuring an adequate path for radiation along the interrogation region; and
   (c) at least one outlet for the exit of the labelled sample and focusing fluids.
2. The microfluidic device according to aspect 1, wherein the at least one waveguide for signal detection is placed at a 45 or at a 135 degree angle with respect to the at least one waveguide for sample excitation.
3. The microfluidic device according to aspect 1, wherein the at least one waveguide for labelled fluid sample excitation and the at least one waveguide for signal detection are optical fibres.
4. An *in vitro* method for the detection of circulating tumour cells (CTCs) in a fluid sample, wherein the method comprises the steps of:
   (a) incubating the fluid sample with a labelling solution wherein the labelling solution comprises a first labelled probe targeting a first CTCs surface marker and at least a second labelled probe targeting a second CTCs surface marker;
   (b) removing the excess of any labelled probe not bound to the CTCs, thereby obtaining a labelled fluid sample; and
   (c) detecting in a microfluidic device according to any one of aspects 1 to 3 a first signal corresponding to the first labelled probe and at least a second signal corresponding to the at least second labelled probe, in the labelled fluid sample;
   wherein the detection of the first signal and the second signal is indicative of the presence of CTCs in the fluid sample;
   wherein the first and second probes are labelled with a different label;
   wherein the first and second labels are independently selected from the group consisting of a fluorescent label, an elastic radiation label, and a Raman label; and
   wherein the first and second probes are independently selected from the group consisting of an antibody, an antibody derivative, a lectin and an aptamer.
5. An *in vitro* method for the detection of circulating tumour cells (CTCs) in a fluid sample, wherein the method comprises the steps of:
   (a) incubating the fluid sample with a labelling solution wherein the labelling solution comprises a first labelled probe targeting a first CTCs surface marker and at least a second labelled probe targeting a second CTCs surface marker, wherein the first and second probes are fluorescently labelled; or alternatively incubating the fluid sample with a labelling solution wherein the labelling solution comprises a first labelled probe targeting a first CTCs surface marker, wherein the first probe is fluorescently labelled;
   (b) removing the excess of any labelled probe not bound to the CTCs, thereby obtaining a labelled fluid sample; and
   (c) detecting in a microfluidic device according to any one of aspects 1 to 3 a first signal corresponding to the first labelled probe and at least a second signal selected from the group consisting of the at least second labelled probe, autofluorescence, and combinations thereof, in the labelled fluid sample;
   wherein the detection of the first signal and the second signal is indicative of the presence of CTCs in the fluid sample; wherein the first and second probes are labelled with a different label; wherein the first and second labels are independently selected from the group consisting of a fluorescent label, an elastic radiation label, and a Raman label; and wherein the first and second probes are independently selected from the group consisting of an antibody, an antibody derivative, a lectin and an aptamer.
6. The method according to any one of aspects 4 or 5, wherein the first and second probes are antibodies.
7. The method according to any one of aspects 4 to 6, wherein the first probe targets a first specific marker for a cell of non-haematological lineage, and the second probe targets a second specific marker for a cell of non-haematological lineage,
   wherein the specific marker for a cell of non-haematological lineage is selected from the group consisting of a epithelial lineage marker, a mesenchymal lineage marker, a stem cell lineage marker and a specific cancer marker.
8. The method according to aspect 7, wherein the first probe targets a specific marker for a cell of epithelial lineage, and/or the second probe targets a specific cancer marker.
9. The method according to aspect 8, wherein the specific marker for a cell of epithelial lineage is selected from the group consisting of EpCAM, CDH1, cytokeratin, MAL, TGFβ-R1, TNF-R1, Ki1, CEA, PSA, CD31, FGFR2, FGFR3, CD44, PSGL1, PD-L1, I-CAM, V-CAM, alpha-V beta-3, alpha-V beta-5, alpha-V beta-6, alpha-VI beta-4, alpha-II beta-1, alpha-R beta-1, alpha-V beta-1, WNT, CD24, Sialyl Lewis A, Sialyl Lewis X, T antigen, Tn antigen, Syalil Tn, GD2, GD3, GD1a, MUC1, MUC2, MUC3, MUC4, MUC5AC and MUC5B;
   and/or wherein the specific cancer marker is selected from a breast cancer marker, a prostate cancer marker, a lung cancer marker, and a colon cancer marker;
10. The method according to any one of aspects 4 to 9, wherein the first probe targets the epithelial cell adhesion molecule (EpCAM), and the second probe targets the receptor tyrosine-protein kinase HER2.
11. An *in vitro* method for diagnosing a tumour and/or metastasis in a subject, wherein the method comprises the step of
   detecting the presence of CTCs in a fluid sample from said subject by a method according to any one of aspects 4 to 10;
   wherein the detection of the presence of CTCs in the fluid sample is indicative that the subject suffers from a tumour and/or metastasis.
12. An *in vitro* method for quantifying circulating tumour cells (CTCs) in a fluid sample, wherein the method comprises the steps of:
   (a) detecting the presence of CTCs in a fluid sample from said subject by a method according to any one of aspects 4 to 10; and
   (b) quantifying the number of CTCs present in the fluid sample from said subject.
13. An *in vitro* method for determining the prognosis of a subject suffering from a tumour and/or metastasis, wherein the method comprises the steps of:
   (a) detecting the presence of CTCs in a fluid sample from said subject by a method according to any one of aspects 4 to 10;
   (b) quantifying the number of CTCs present in the fluid sample from said subject; and
   (c) comparing the number of CTCs present in the fluid sample from said subject with the number of CTCs present in a reference sample;
   wherein an increase in the number of CTCs in the fluid sample with respect to the number of CTCs in the reference sample is indicative that the subject has a poor prognosis, or
   wherein a decrease in the number of CTCs in the fluid sample with respect to the number of CTCs in the reference sample is indicative that the subject has a good prognosis.
14. An *in vitro* method for monitoring the progression of a tumour and/or metastasis in a subject, wherein the method comprises the steps of:
   (a) detecting the presence of CTCs in a fluid sample from said subject by a method according to any one of aspects 4 to 10;
   (b) quantifying the number of CTCs present in the fluid sample from said subject; and
   (c) comparing the number of CTCs present in the fluid sample from said subject with the number of CTCs present in a reference sample obtained from the same subject at an earlier time point of the disease;
   wherein an increase in the number of CTCs in the fluid sample with respect to the number of CTCs in the reference sample is indicative of tumour progression, or
   wherein a decrease in the number of CTCs in the fluid sample with respect to the number of CTCs in the reference sample is indicative of tumour regression or remission.
15. An *in vitro* method for monitoring the response to a therapy in a subject suffering from cancer and/or metastasis, wherein the method comprises the steps of:
   (a) detecting the presence of CTCs in a fluid sample from said subject obtained after the therapy is administered, said detection carried out by a method according to any one of aspects 4 to 10;
   (b) quantifying the number of CTCs present in the fluid sample from said subject obtained after the therapy is administered; and
   (c) comparing the number of CTCs present in the fluid sample from said subject with the number of CTCs present in a reference sample obtained from the same subject before the therapy is administered;
   wherein an increase in the number of CTCs in the fluid sample with respect to the number of CTCs in the reference sample is indicative that the subject does not respond to therapy, or
   wherein a decrease or a lack of change in the number of CTCs in the fluid sample with respect to the number of CTCs in the reference sample is indicative that the subject responds to therapy.
16. The method according to any one of aspects 11 to 15, wherein the tumour is a breast tumour, more preferably a HER2 positive breast tumour, and preferably a metastatic breast tumour.
17. A kit comprising:
   (a) a microfluidic device according to any one of aspects 1 to 3, and
   (b) a first labelled probe targeting a first CTCs surface marker and, optionally,
   at least a second labelled probe targeting a second CTCs surface marker wherein the first and second probes are labelled with a different label and wherein the first and second labels are independently selected from the group consisting of a fluorescent label, an elastic radiation label, and a Raman label; and
   wherein the first and second probes are independently selected from the group consisting of an antibody, an antibody derivative, a lectin and an aptamer.

### EXAMPLES

The invention is described below by means of the following examples which are merely illustrative and by no means limiting for the scope of the invention.

### Materials and Methods

**Fabrication of PDMS microfluidic chips.** 3" Si wafers were used as a substrate to produce the SU8 (SU8-2150, MicroChem Corp) structures. Prior to SU8 spin coating, the Si wafer is thoroughly dehydrated in a hot plate at 200°C for two hours. An adhesion promoter (OmniCoat, MicroChem Corp.) was then applied over the bare Si surface and then baked on a hotplate at 200°C for one minute. The wafer was kept at 100°C on a hot plate to avoid any hydration of the substrate. SU8 masters were fabricated employing a standard technique widely used during master construction for PDMS. Microfluidic chip pattern was engraved using a submicron resolution UV laser lithography (DWL 66fs from Heidelberg Instruments) on a 4 inches chromium photomasks. For a single chip elaboration two moulds were constructed, one containing the structures for the base of the chip, microfluidic channels and grooves for the optical fibres, and another one from which the channels of the cover, devoted to vertical sample flow focusing, were cast. The width of the optical fibres grooves was set during the photomask elaboration at 120 µm -5 µm below the diameter of the stripped optical fibre- to ensure a proper grip between the fibre cladding and the PDMS. Care was put during the construction of the photomask so that, after the realization of the SU8 master, the channels containing the pumping fibres and the collection fibres would be aligned in such a way that the axis of the fibres would intersect exactly in the middle of the microfluidic channel once they were positioned. For the spin-coating process, 2 ml of SU8 were dispensed over a dehydrated 3 inches Si wafer and then spin-coated at 2800 RPM for 40 seconds with a 500 RPM/s ramp. These parameters allowed to obtain a SU8 thickness of approximately 120-130 µm; the required to prevent fibres from protruding from the PDMS groove. The wafer with the SU8 layer was then soft-baked for 1 h at 100°C. Once the soft-bake finished and the solvent from the SU8 was completely evaporated, an edge bead removal (EBD) of the SU8 spin coated layer was performed. This process allows the photomask to be in close contact with the baked SU8 exposed surface during the UV exposure, minimising UV light diffraction effects in the patterned mask contours. The UV exposure of the SU8 was performed with a mercury discharge lamp with mask aligner mounting (MG 1410, Karl Suss). The optimal exposure time was found to be 4.5 seconds for a density power of 124 mW/cm2 for a reference wavelength of 405 nm. After the SU8 has been exposed it follows a post exposure bake at 65°C for 5 min and 100°C for 10 min. The hot plate is then switched off and allowed to cool down to room temperature. The Si wafer is then developed in PGMEA solvent (AZ® EBR Solvent) multiple times until the SU8 structures are exposed and no uncrosslinked SU8 residue is visible on the Si wafer. An oxygen plasma exposure was applied to the finished master to eliminate the exposed Omnicoat layer and leave a cleaner Si surface. A reactive-ion etching (RIE) (PlasmaPro NGP80, Oxford Instruments) was used for that purpose and the conditions were: power: 100 W, oxygen flow rate: 35 sccm, pressure: 100 mTorr and time: 1 min.

**PDMS bonding and optical fibre positioning.** The SU8 masters were placed at the bottom of an aluminium foil container with the face with the SU8 structures pointing upwards and the degassed PDMS (Sylgard 184, Dow Corning) was later poured into the container and left to cure at 80°C for 2 hours. After curing, the PDMS slabs were then thoroughly rubbed with optical cleaning tissues soaked in acetone and rinsed with ethanol. The PDMS surfaces were then irreversibly bonded together exposing the surfaces to a RIE generated oxygen plasma under the following conditions: power: 10 W, oxygen flow rate: 50 sccm, pressure: 80 mTorr and time: 20 s. Figure 1 shows an ESEM image of both interrogation zones with pumping and collecting fibres inserted for illustration purposes. A total of four single-ended FC connectorised optical fibre patch cords were inserted in a single chip. To ensure a proper illumination and a clean diverging Gaussian beam in the interrogation zone, the fibres were cleaved and later observed under the microscope to verify a clean cut of the face of the fibre before inserting them by hand in the dedicated grooves entrances located at both sides of the chip. Likewise, flat and homogeneous fibre cleaves guaranteed an optimal light harvesting for the collection fibres. Each groove end was wetted with a droplet of ethanol which entered inside the groove by capillarity. This reduced the friction and helped during the process of fibre insertion.

**Sample measurement with the optofluidic chip.** The optofluidic chip was placed under an inverted microscope (IN480TC-FL, AMScope) to obtain clear images of the focusing of the fluid. Two pressure controllers (OB1-mk3, Elveflow) were employed to pump the fluid inside the chip; one channel for each of the four sacrificial fluids -two for vertical focusing and two more for horizontal focusing- and one channel for the sample flow. The independent treatment of each input flow allowed not only to adjust the area of the cross-section of the centrally focused flow but also the position of the focused flow within the microfluidic channel with respect to the walls of the central microfluidic channel. The pumping light for both independent interrogation zones was generated by a laser diode (473nm 06-01 series, Cobolt) coupled to a 50:50 1x2 optical fibre splitter (FC488-50B-APC-1, Thorlabs). Each splitter end was mated to both single mode input optical fibres (3.5 µm core) of the chip. Typical pumping powers inside the microfluidic channel during the measurement process were of the order of 10 mW. The two-output fluorescence collection multimode patch fibres of the microfluidic chip were directly connected to two photomultiplier tubes (R928, Hamamatsu) enclosed in an aluminium housing (PXT1/M, Thorlabs) using an FC connector. To avoid possible high pumping light intensities from reaching the photomultiplier (originated mainly from scattered light in the cells) two 500 nm cut-off wavelength longpass filters (FELH0500, Thorlabs) were located in the aluminium housing between the optical fibre tip and the photocathode. Additional bandpass filters (86-988 and 33-330 from Edmund Optics) were placed next to each longpass filters to allow the selected transmission of light at each photomultiplier detector. Both photomultiplier tubes were directly connected to an oscilloscope (TDS 1012B from Tektronix) to visualize fluorescence bursts simultaneously while the measuring process was taking place. The internal resistance of the oscilloscope (1 M Ω) was used as the output impedance. A two-channel home-made voltage follower with a saturation voltage of 9 V was then applied to the voltage signal from the oscilloscope and a data acquisition card (NI USB-6212 BNC, National Instruments) was used in order acquire the signal data from both channels. The low impedance in the voltage follower prevented from ghosting to appear in one of the channels, especially at high sampling rates in the DAQ. To minimise any noise from electronic equipment coaxial cables were employed to connect all the electronic components of the setup. Data acquisition from the signal was accomplished by using a Lab-VIEW program that allowed to simultaneously save the data from the two channels to be later treated using Scilab software for correlation measurements of the two signals. Sample measurement with the flow-cytometer: Flow cytometry of the samples was carried out in a NovoCyte Flow Cytometer (from AceaBiosciences), equipped with a 488 nm excitation laser and 530/30 nm and 675/30 nm detection filters. Cytometric data were analysed with NovoExpress and FloJo VX software.

**Cell Culture.** AU-565 cancer cells derived from mammary breast expressing HER2 and EpCAM receptors, and RAMOS, HER2, EpCAM negative, were obtained from the American Tissue Culture Collection (ATCC, Manassas, VA, USA) and cultured in RPMI media supplemented with 10% foetal bovine serum, at 37°C in a 5% CO₂ atmosphere.

**Fluorophore characterisation.** Absorption and emission profiles for FITC and PerCP/Cy5.5 conjugates were collected with a UV-visible spectrophotometer Evolution 201 (ThermoScientific) and Cary Eclipse Fluorescence Spectrophotometer (Agilent Technologies).

**Cell Labelling for microscopy analysis.** AU-565 cells were harvested from culture dishes with trypsin/EDTA 0.25% and seeded in 8-well µ-plate Ibidi at 1x10⁴ cells/well, for 24 h at 37 °C in a 5% CO₂ atmosphere. Following day, cells were washed with PBS and fixed with 4% paraformaldehyde in PBS for 30 min. Then, cells were treated with 1% NP40 in PBS for 15 min at 37°C. 2.0 µg/mL of mouse anti-human her2 FITC-conjugated antibody (Abcam) and 2.0 µg/mL of mouse anti-human EpCAM PerCP/Cy5.5-conjugated antibody (Abcam) were added to AU-565 cells for 1h of incubation at 37°C. Finally, cells were washed three times with PBS and 100 nM DAPI was added for nuclei staining. Cells were analysed by laser scanning confocal microscopy using 488 nm excitation laser and 540/30 nm and 650LP nm detection filters, to collect fluorescence from FITC and PerCP/Cy5.5, respectively. DAPI fluorescence was collected with epi-fluorescence filters and images were processed with ImageJ software.

**Cell Labelling for microfluidics and flow cytometry. Cells were harvested from** culture dishes with EDTA 0.25% PBS dissociation buffer and 1x10⁶ cells were placed in suspension in PBS 10% FBS. For each 100 µL of cell suspension, with the appropriate AU-565:RAMOS ratio, 4 µL of mouse anti-EpCAM-PerCP/Cy5.5 and 10 µL of mouse anti-HER2-FITC were added. Then, cells were washed by centrifugation at 170g for 5 min and suspended in cold-PBS. Samples were measured in the optofluidic device and the flow-cytometer.

**Clinical samples.** Personal and clinical data were recorded according to standard clinical procedures. All specimens were obtained with informed consent. The study was approved by the local Ethics and Clinical Research Committee. 8 ml of blood was drawn in 10 ml vacutainer tubes containing EDTA and processed in the first 24 h. Results for CTCs were linked to clinical data.

**Preparation of clinical samples.** Blood samples from cancer and healthy patients were obtained from the Servicio de Oncologia Clinica of the HM Hospital Universitario Torrelodones-Madrid. Peripheral blood mononuclear cells (PBMCs) were isolated from the whole blood using Ficoll-Paque PLUS (purchased from GE Healthcare Life Science). 15 mL of Ficoll solution was added to a 50 mL Leucosep Centrifuge tubes (Greiner Bio One) and blood was disposed as a layer onto Ficoll. Samples were centrifuged at 400g for 40 min at 18°C, and the resulting PBMCs layer was separated from the rest of phases. PMBCs were washed twice in 10 mL HBSS by centrifugation at 100g for 5 min and finally suspended in RPMI-1640 supplemented with 10% FBS, until use. Then, 1x10⁷ cells were pelleted and placed in 100 µL PBS 10% FBS and 4 µL of anti-EpCAM-PerCP/Cy5.5 and 10 µL of anti-HER2-FITC were added. After 1h at RT, cells were washed by centrifugation at 500g for 5 min and suspended in cold-PBS. Same procedures were following for the measurement of clinical samples in the optofluidic device and the cytometer, as mentioned above.

### Example 1: Test performance of the microfluidic chip of the invention

In a specific embodiment, the microfluidic chip of the invention comprises a 3D hydrodynamic focusing chip (Figure 1A). The fluid containing the target sample is injected in one of the channels while a sheath flow is injected at the same time through the adjacent channels. Due to the involved low time scales and the magnitude of the diffusion rate in a typical experiment, sheath and sample flows do not mix but confines the sample flow to the central region of the channel (Figure 1B). Two interrogation zones, placed with a separation of 1 mm, are implemented in the chip. Each of them comprises a pumping fibre (473 nm)-oriented perpendicularly to the microfluidic channel where the focused sample is constrained- and a collecting fibre to collect either the red or green fluorescence. The collecting fibres are oriented at a 45° angle respect to the excitation source to avoid collecting the light from the pumping source. Single mode fibres are selected for excitation (473 nm) to obtain an interrogation volume of the order of the size of the cells (Figure 1C). Fluorescence emission collection is accomplished by means of two 62.5 µm core high numerical aperture (NA = 0.275) multimode optical fibres, both for the red and the green fluorescence. Such a big core and NA allows obtaining an uninterrupted fluorescence signal from the cells as they flowed through the length of the pumping volume.

To test the performance of the device the inventors selected two cell lines, AU-565, HER2 positive/EpCAM positive, and RAMOS, HER2 negative/EpCAM negative. Thus, to identify the positive cells the inventors employed anti-HER2 and anti-EpCAM antibodies, labelled with fluorescein isothiocyanate (FITC) and peridinin-chlorophyllprotein cyanin-5.5 (PerCP/Cy5.5), respectively. These two dyes (Figure 2A) were selected as the fluorescent reporters due to their absorption properties in the blue range, which fit perfectly with the optical fibres excitation wavelength (473 nm) and their emission bands in the visible at green (532 nm, FICT) and red (676 nm, PerCP/Cy5.5). Confocal laser scanning microscopy (Figure 2B) reveals a successful staining of AU-565 cells, where HER2-FITC is presented in green and EpCAM-PerCP/Cy5.5 is in red. AU-565 were mixed with RAMOS at different ratios (1:1, 1:10, 1:100 and 1:1000) to demonstrate the capability of the optofluidic chip to detect fluorescence-highlighted cells, even at low concentration. For comparison, standard flow cytometry was carried out in parallel on the same samples.

Figure 3A shows a short record of the fluorescence signals obtained for a sample with 1:10 positive:negative cell ratio. Red signals correspond to the epithelial receptors (EpCAM) while green is assigned to the HER2. Although the figure shows the normalized intensity of both signals, those of the green channel are larger than those from the red due to the higher quantum yield of FICT as compared with PerCP/Cy5.5. Notably, this graph shows positive events for green, red and for both. Knowing that there is no correlation between apparently random events, in the case of detection of a positive event (an AU-565 cell), there must exist redundancy in the obtained data (Figure 3B) as both interrogation zones are exact copies. To automatically consider two signals (red and green) associated with the same cell, the time shift between the red and green channels signals is monitored employing a correlation measurement (Figure 3C). Then, when a green signal is detected the closest red peaks are selected and their elapsed time compared. If the temporal distance between both peaks fits with the adequate time shift, they are considered correlated. Results for a given sample can then be represented as a function of their green and red intensities. Figures 3D and 3E show the results obtained by measuring a 1:1000 positive:negative cell ratio with the optofluidic chip and a flow cytometer, respectively. Flow cytometry reveals a counting of 43 AU-565 cells (expressing EpCAM and HER2 receptors) and 21163 total cells, evidencing a ratio of 2.04:1000 (slightly different from the theoretical one due to experimental errors). When detecting with the optofluidic chip, 54 malignant and 22765 normal cells were identified. This corresponds to a 2.37:1000 ratio, which is in good agreement with the result observed for the standard cytometry.

Notably, the optofluidic device produces a considerable larger number of green events than flow cytometry, this is because, under 473 nm pumping, the auto fluorescence of the cell is mainly in the green part of the spectrum and may overlap with the fluorescence emission of the cells marked with FITC. Similar correspondence was observed, as well, for the rest of measured samples (Figure 3G), validating the feasibility of the method for further application in real samples.

### Example 2: Use of the microfluidics chip and method for the analysis of healthy donor and patient samples

Between January and September of 2016, 3 healthy donors and 5 patients were enrolled. All the patients were diagnosed with metastatic breast cancer but with secondary tumours in different locations. Their clinical characteristics and the state of cancer evolution at the time of blood extraction are summarized in Table 1. Figure 4 includes the computerized tomography data of the patients at the time of blood extraction. Notably, previous data had been also added to patient 2 as she is actually in complete remission. Personal and clinical data were recorded according to standard clinical procedures. All patients provided written informed consent before study enrolment. The study was approved by the local Ethics and Clinical Research Committee.

The presence of circulating tumour cells in real blood samples was evaluated by processing and analysing blood from the different healthy donors and the breast cancer patients. To reduce the volume of the initial sample (8 mL), only the peripheral blood mononuclear cells (PBMCs) were collected, stained, and run into flow cytometry and the optofluidic chip. Healthy blood, without the presence of circulating tumour cells, was first run to establish a threshold which categorizes as negative events due to the normal PBMCs (Figures 5A and 5C). Contrary to the observed in cellular lines, for both, flow-cytometry and microfluidics, no spurious EpCAM positive was observed. No matter, the amount of HER2 positive events was notably larger. In the case of the flow-cytometer this increase in the number of HER2 positives are ascribed to expression, although low, of HER2 receptors in the NK/granulocyte population. For the optofluidic chip, this last reason must be added to the background auto-fluorescence collection with the green channel. Thus, we assume as a positive event, detected cells above the established thresholds for both dyes, green (antiHER2) and red (antiEpCAM). Flow cytometry and optofluidic measurements obtained for the cancer patients are shown in Figures 5B and 5C. CTCs were quantified in similar amounts, with both methods, for four of the five patients but with one presenting none (CP2). These data correlate well with those obtained with conventional diagnosis at the sample extraction (Table 1 and Figure 4). First, samples which show a low CTC count, CP1 and CP5, present a stabilized lung metastasis (CP1) and a malignant pleural effusion and mediastinal mass (CP5), which is still in progression but after four cycles of specific treatment. In contrast, CP4 presents an osteoblastic metastasis in progression and CP3, the patient with a larger number of CTCs, was recently diagnosed with progression and is already initiating treatment. Notably, CP2 which shows no evidence of CTCs in her blood, is presently in a complete remission of her previously diagnosed C6 and other bone lytic and visceral metastasis.

**Table 1: Clinical characteristics and the state of cancer progression at the time of blood extraction. F, Female; ER, estrogen receptor; PR, progesterone receptor.**

| **Patient** | **Age (years)/sex** | **ER/PR** | **Metastasis** | **Disease Status** |
|---|---|---|---|---|
| CP1 | 68/F | Positive | Visceral | Stable disease |
| CP2 | 41/F | Negative | Bone | Complete remission |
| CP3 | 59/F | Positive | Lymph node | Progression |
| CP4 | 44/F | Negative | Bone | Progression |
| CP5 | 49/F | Positive | Visceral | Progression |

## Claims

1. A microfluidic device for the detection of circulating tumour cells (CTCs) in a fluid sample comprising:
(a) a cell focusing element selected from a hydrodynamic focusing element and an inertial focusing element, wherein
the hydrodynamic focusing element comprises at least three inlets, a first central inlet for the entrance of a labelled fluid sample, and a second and third inlets for the entrance of a focusing fluid for flow focusing of the labelled fluid sample in a first plane, wherein the second and third inlets are situated at opposite sides of the first central inlet, and optionally, a fourth and fifth inlets for the entrance of a focusing fluid for flow focusing of the labelled fluid sample in a second plane perpendicular to the first plane, wherein the fourth and fifth inlets are situated at opposite sides of the first central inlet, all the inlets converging into a single microfluidic channel; and wherein
the inertial focusing element comprises a functional microchannel selected from the group consisting of a straight channel, a straight channel with pillar arrays, a straight channel with expansion-contraction arrays, a spiral channel, a serpentine channel, and combinations thereof;
(b) a single microfluidic channel comprising at least two interrogation regions, wherein each interrogation region comprises at least one waveguide for labelled fluid sample excitation, and at least one waveguide for signal detection, wherein the at least one waveguide for signal detection is not placed at a 180 degree angle with respect to the at least one waveguide for sample excitation, wherein the microfluidic channel optionally comprises lenses and/or mirrors for ensuring an adequate path for radiation along the interrogation region; and
(c) at least one outlet for the exit of the labelled sample and focusing fluids.

2. The micro fluidic device according to claim 1, wherein the at least one waveguide for signal detection is placed at a 45 or at a 135 degree angle with respect to the at least one waveguide for sample excitation.

3. The micro fluidic device according to claim 1, wherein the at least one waveguide for labelled fluid sample excitation and the at least one waveguide for signal detection are optical fibres.

4. An *in vitro* method for the detection of circulating tumour cells (CTCs) in a fluid sample, wherein the method comprises the steps of:
(a) incubating the fluid sample with a labelling solution wherein the labelling solution comprises a first labelled probe targeting a first CTCs surface marker and at least a second labelled probe targeting a second CTCs surface marker;
(b) removing the excess of any labelled probe not bound to the CTCs, thereby obtaining a labelled fluid sample; and
(c) detecting in a microfluidic device according to any one of claims 1 to 3 a first signal corresponding to the first labelled probe and at least a second signal corresponding to the at least second labelled probe, in the labelled fluid sample;
wherein the detection of the first signal and the second signal is indicative of the presence of CTCs in the fluid sample;
wherein the first and second probes are labelled with a different label;
wherein the first and second labels are independently selected from the group consisting of a fluorescent label, an elastic radiation label, and a Raman label; and
wherein the first and second probes are independently selected from the group consisting of an antibody, an antibody derivative, a lectin and an aptamer.

5. An *in vitro* method for the detection of circulating tumour cells (CTCs) in a fluid sample, wherein the method comprises the steps of:
(a) incubating the fluid sample with a labelling solution wherein the labelling solution comprises a first labelled probe targeting a first CTCs surface marker and at least a second labelled probe targeting a second CTCs surface marker, wherein the first and second probes are fluorescently labelled; or alternatively incubating the fluid sample with a labelling solution wherein the labelling solution comprises a first labelled probe targeting a first CTCs surface marker, wherein the first probe is fluorescently labelled;
(b) removing the excess of any labelled probe not bound to the CTCs, thereby obtaining a labelled fluid sample; and
(c) detecting in a microfluidic device according to any one of claims 1 to 3 a first signal corresponding to the first labelled probe and at least a second signal selected from the group consisting of the at least second labelled probe, autofluorescence, and combinations thereof, in the labelled fluid sample;
wherein the detection of the first signal and the second signal is indicative of the presence of CTCs in the fluid sample; wherein the first and second probes are labelled with a different label; wherein the first and second labels are independently selected from the group consisting of a fluorescent label, an elastic radiation label, and a Raman label; and wherein the first and second probes are independently selected from the group consisting of an antibody, an antibody derivative, a lectin and an aptamer.

6. The method according to any one of claims 4 or 5, wherein the first and second probes are antibodies.

7. The method according to any one of claims 4 to 6, wherein the first probe targets a first specific marker for a cell of non-haematological lineage, and the second probe targets a second specific marker for a cell of non-haematological lineage, wherein the specific marker for a cell of non-haematological lineage is selected from the group consisting of a epithelial lineage marker, a mesenchymal lineage marker, a stem cell lineage marker and a specific cancer marker.

8. The method according to claim 7, wherein the first probe targets a specific marker for a cell of epithelial lineage, and/or the second probe targets a specific cancer marker.

9. The method according to claim 8, wherein the specific marker for a cell of epithelial lineage is selected from the group consisting of EpCAM, CDH1, cytokeratin, MAL, TGFβ-R1, TNF-R1, Ki1, CEA, PSA, CD31, FGFR2, FGFR3, CD44, PSGL1, PD-L1,I-CAM, V-CAM, alpha-V beta-3, alpha-V beta-5, alpha-V beta-6, alpha-VI beta-4, alpha-II beta-1, alpha-R beta-1, alpha-V beta-1, WNT, CD24, Sialyl Lewis A, Sialyl Lewis X, T antigen, Tn antigen, Syalil Tn, GD2, GD3, GD1a, MUC1, MUC2, MUC3, MUC4, MUC5AC and MUC5B;
and/or wherein the specific cancer marker is selected from a breast cancer marker, a prostate cancer marker, a lung cancer marker, and a colon cancer marker;

10. The method according to any one of claims 4 to 9, wherein the first probe targets the epithelial cell adhesion molecule (EpCAM), and the second probe targets the receptor tyrosine-protein kinase HER2.

11. An *in vitro* method for diagnosing a tumour and/or metastasis in a subject, wherein the method comprises the step of
detecting the presence of CTCs in a fluid sample from said subject by a method according to any one of claims 4 to 10;
wherein the detection of the presence of CTCs in the fluid sample is indicative that the subject suffers from a tumour and/or metastasis.

12. An *in vitro* method for quantifying circulating tumour cells (CTCs) in a fluid sample, wherein the method comprises the steps of:
(a) detecting the presence of CTCs in a fluid sample from said subject by a method according to any one of claims 4 to 10; and
(b) quantifying the number of CTCs present in the fluid sample from said subject.

13. An *in vitro* method for determining the prognosis of a subject suffering from a tumour and/or metastasis, wherein the method comprises the steps of:
(a) detecting the presence of CTCs in a fluid sample from said subject by a method according to any one of claims 4 to 10;
(b) quantifying the number of CTCs present in the fluid sample from said subject; and
(c) comparing the number of CTCs present in the fluid sample from said subject with the number of CTCs present in a reference sample;
wherein an increase in the number of CTCs in the fluid sample with respect to the number of CTCs in the reference sample is indicative that the subject has a poor prognosis, or
wherein a decrease in the number of CTCs in the fluid sample with respect to the number of CTCs in the reference sample is indicative that the subject has a good prognosis.

14. The method according to any one of claims 11 to 13, wherein the tumour is a breast tumour, more preferably a HER2 positive breast tumour, and preferably a metastatic breast tumour.

15. A kit comprising:
(a) a microfluidic device according to any one of claims 1 to 3, and
(b) a first labelled probe targeting a first CTCs surface marker and, optionally,
at least a second labelled probe targeting a second CTCs surface marker wherein the first and second probes are labelled with a different label and wherein the first and second labels are independently selected from the group consisting of a fluorescent label, an elastic radiation label, and a Raman label; and
wherein the first and second probes are independently selected from the group consisting of an antibody, an antibody derivative, a lectin and an aptamer.
